# FASCICULE DE BREVET EUROPEEN

(11) **EP 2 699 545 B1**
(45) Date de publication et mention de la délivrance du brevet: **25.05.2016**
(21) Numéro de dépôt: 12722423.6
(22) Date de dépôt: 17.04.2012
(51) Int. Cl.: C07D 207/22, C07D 403/04, A61K 31/40, A61K 31/403, A61P 3/00

(54) **NOUVEAUX DERIVES AMINO-PYRROLINIQUES, LEUR UTILISATION DANS LA PREVENTION ET/OU LE TRAITEMENT DU SYNDROME METABOLIQUE**
NEUE AMINOPYRROLINDERIVATE UND IHRE VERWENDUNG ZUR VORBEUGUNG UND/ODER BEHANDLUNG VON METABOLISCHEM SYNDROM
NOVEL AMINO-PYRROLINE DERIVATIVES, AND USE THEREOF IN THE PREVENTION AND/OR TREATMENT OF METABOLIC SYNDROME

(30) Priorité: 19.04.2011 FR 1153375; 13.02.2012 FR 1251302
(43) Date de publication de la demande: 26.02.2014
(73) Titulaire: Université de Strasbourg, 67000 Strasbourg (FR)
(72) Inventeur: BOUSQUET, Pascal, F-67000 Strasbourg (FR); EHRHARDT, Jean Daniel, F-67370 Frankenheim (FR); FELLMANN, Lyne, F-67000 Strasbourg (FR); GASPARIK, Vincent, F-67100 Strasbourg (FR); GRENEY, Hugues, F-67100 Strasbourg (FR); HADJERI, Mohamed, 38090 Villefontaine (FR); MANN, André, F-67540 Ostwald (FR); NIEDERHOFFER, Nathalie, F-67150 Uttenheim (FR); SCHANN, Stephan, F-67400 Illkirch (FR)
(74) Mandataire: Gallois, Valérie
(86) Numéro de dépôt international: PCT/FR2012/050835
(87) Numéro de publication internationale: WO 2012/143660

(56) Documents cités:
- WO-A1-2011/044229
- WO-A1-2011/050030
- US-A- 3 563 994
- US-A- 4 533 739
- US-A- 4 579 951
- FRED M HERSHENSON ET AL: "Synthesis and Antihypertensive Activity of 2-Arylamino-1-azacycloalkenes", JOURNAL OF MEDICINAL CHEMISTRY, AMERICAN CHEMICAL SOCIETY, US, vol. 14, no. 10, 1 octobre 1971 (1971-10-01), pages 907-909, XP002622490, ISSN: 0022-2623, DOI: 10.1021/JM00292A003
- DE JONG A P ET AL: "Relationships between structure and alpha-adrenergic receptor affinity of clonidine and some related cyclic amidines", EUROPEAN JOURNAL OF PHARMACOLOGY, ELSEVIER SCIENCE, NL, vol. 69, no. 2, 16 janvier 1981 (1981-01-16), pages 175-188, XP025547328, ISSN: 0014-2999, DOI: 10.1016/0014-2999(81)90412-X [extrait le 1981-01-16]
- UROSEVIC DRAGAN ET AL: "LNP 906, the first high-affinity photoaffinity ligand selective for I1 imidazoline receptors", BRITISH JOURNAL OF PHARMACOLOGY, NATURE PUBLISHING GROUP, BASINGSTOKE, HANTS; GB, vol. 142, no. 3, 1 juin 2004 (2004-06-01), pages 609-617, XP002619045, ISSN: 0007-1188, DOI: 10.1038/SJ.BJP.0705784 [extrait le 2009-01-29]
- VAN ZWIETEN P A: "Antihypertensive drugs interacting with central imidazoline (I1)-receptors.", EXPERT OPINION ON INVESTIGATIONAL DRUGS NOV 1998 LNKD- PUBMED:15991929, vol. 7, no. 11, novembre 1998 (1998-11), pages 1781-1793, XP002659124, ISSN: 1744-7658

## Description

La présente invention concerne des nouveaux dérivés amino-pyrroliniques, leur utilisation dans la prévention et/ou le traitement du syndrome métabolique.

Le syndrome métabolique, encore appelé syndrome X ou syndrome dysmétabolique, est constitué d'un ensemble de symptômes cardiovasculaires, notamment l'hypertension artérielle, et métaboliques (hypercholestérolémie, résistance à l'insuline, intolérance au glucose, obésité abdominale) (Reaven, G,M. 1988. Banting lecture 1988; Role of insulin résistance in human disease. Diabetes 37:1595-1607). La réunion de tous ces symptômes est unanimement reconnue comme constituant un facteur de risques cardiovasculaires et métaboliques majeur. Les complications de ce syndrome métabolique sont notamment l'athérosclérose, la maladie coronaire, l'insuffisance rénale, l'insuffisance cardiaque, le diabète, l'obésité. La prévalence de ce facteur de risque est encore variable selon les pays. Elle tend néanmoins à s'établir partout dans le monde autour de 30% de la population générale. Son développement rapide est extrêmement important et pose des problèmes de santé publique majeurs.

La thérapeutique médicamenteuse actuelle du syndrome métabolique est constituée d'associations médicamenteuses composées de médicaments n'agissant chacun que sur un élément du syndrome métabolique qui en comporte entre 3 et 6: médicaments antihypertenseur (parfois plusieurs associés) en combinaison avec des médicaments hypoglycémiant (parfois plusieurs associés) en combinaison avec des médicaments hypolipémiants (parfois plusieurs associés).

Ces combinaisons sont génératrices de nombreux effets indésirables et certaines, comme les statines, sont très coûteuses.

Bien que plusieurs études aient montré que le syndrome métabolique est associé à une hyperactivité sympathique mesurée par une augmentation de l'Activité Nerveuse Sympathique Musculaire (ANSM) et ce, même en l'absence d'hypertension artérielle (Huggett R. J.; Burns J.; Mackintosh A.F.; Mary D.A.S.G. Sympathetic Neural Activation in Nondiabetic Metabolic Syndrome and Its Further Augmentation by Hypertension Hypertension, 2004, 44:847-852), aucun médicament actuellement disponible, apte à inhiber le système sympathique, n'est proposé pour traiter le syndrome métabolique et ses conséquences.

Les récepteurs aux imidazolines (RI) sont impliqués dans plusieurs systèmes biologiques de régulation. Le principal est la régulation, par le système nerveux sympathique, de la pression artérielle (Bousquet, P,; Feldman, J. Drugs Acting on Imidazoline Receptors: a Review of their Pharmacology, their Use in Blood Pressure Control and their Potential Interest in Cardioprotection. Drugs 1999, 58, 799-812; Head, G. A,; Mayorov, D. N. Imidazoline Receptors, Novel Agents and Therapeutic Potential. Cardiovasc. Hematol. Agents Med. Chem. 2006, 4, 17-32).

Ils sont aussi impliqués dans d'autres fonctions physiopathologiques telles que la sécrétion d'insuline (Chan, S. L. F. Clonidine-displacing Substance and its Putative Role in Control of Insulin Secretion : A Minireview. Gen. Pharmacol. 1998, 31, 525-529), la régulation de la pression intraoculaire (Chu, T. C,; R,, S. R,; Ogidigben, M. J,; Potter, E. D. Potential Mechanisms of Moxonidine-induced Ocular Hypotension: Role of Norepinephrine. J. Ocul. Pharmaco/. Ther. 1997, 13, 489-496) et le contrôle de la fréquence cardiaque (Roegel, J. C,; de Jong, W,; Monassier, L,; Feldman, J,; Bousquet, P. Comparative Effects of Idazoxan, Prazocine and Yohimbine on Coronary Ligation-induced Arrhythmias in Spontaneously Hypertensive Rats. J. Cardiovasc. Pharmaco/. 1996, 27, 226-234).

Les RI sont classés en trois sous-types principaux RI₁. RI₂ et RI₃. Le premier sous-type RI₁ est localisé dans la partie rostro-ventrolatérale (RVLM) du tronc cérébral et est impliqué dans la régulation centrale de la fonction cardiovasculaire

Le RI₁ est sensible à la clonidine et à d'autres composés de type imidazoline mais pas aux catécholamines et sont donc différents des récepteurs adrénergiques α₂ (Rα₂A).

L'agmatine, des «substances déplaçant la clonidine» et l'harmane sont des ligands endogènes des RI₁.

Le RI₂ est insensible à la clonidine mais sensible à l'idazoxan. Les RI₂ sont subdivisés en deux sous-types selon leur affinité pour l'amiloride (Tesson, F,; Prib-Buus, C,; Lemoine, A,; Pegorier, J. P,; Parini, A. A Subcellular Distribution of Imidazoline-Guanidinium Receptive Sites in Human and Rabbit Liver. Major Localization to the Mitochondrial Outer Membrane. J. Biol. Chem. 1991, 266, 155-160).

Un troisième sous type, le RI₃, a été ajouté à la classification et est impliqué dans la régulation de l'insuline (Englen, R. M,; Hudson, A. L,; Kendall, D. A,; Nutt, D. J,; Morgan, N. G,; Wilson, V. G,; Dillon, M. P. 'Seeing Through a Glass Darkly' : Casting Light on Imidazoline 'I' Sites. Trends Pharmaco/. Sci. 1998, 19, 381-390).

La clonidine est le composé leader de la première génération des médicaments antihypertenseurs, agissant centralement. Elle se lie à la fois aux RI₁ et aux Rα₂A. Ses effets secondaires, la sédation en particulier, sont clairement dus à l'activation des Rα₂A (De Sarro, G. B,; Ascioti, C,; Froio, F,; Libri, V,; Nistico, G. Evidence that Locus Coeruleus is the Site where Clonidine and Drugs Acting at **α**₁- et **α**₂-Adrenoceptors Affect Sleep et Arousal Mechanisms. Br. J. Pharmacol. 1987, 90, 675-685).

Des médicaments tels que la moxonidine et la rilmenidine ont une sélectivité pour les RI₁ par rapport au Rα₂A car ils possèdent une affinité plus faible pour les Rα₂A et provoquent par conséquent moins d'effets secondaires chez les patients hypertendus.

Jusqu'à récemment, le manque de médicaments hypotenseurs sélectifs des RI a très fortement ralenti la recherche sur ces récepteurs.

D'un côté, les médicaments imidazoliniques hypotenseurs tels que la clonidine et ses analogues sont tous des médicaments « hybrides » puisqu'ils se lient à la fois à RI₁ et à Rα₂A.

D'un autre côté, les quelques composés disponibles jusqu'à présent hautement sélectifs pour RI₁ tels que AGN192403 (Munk, S. A,; Lai, R. K,; Burke, J. E,; Arasasingham, P. N,; Kharlamb, A. B,; Manlapaz, C. A,; Padillo, E. U,; Wijono, M. K,; Hasson, D. W,; Wheeler, L. A,; Garst, M. E. Synthesis and Pharmacologic Evaluation of 2-endo-Amino-3-exo-isopropylbicyclo[2,2,1]heptane: A Potent Imidazoline1 Receptor Specific Agent. J. Med. Chem 1996, 39, 1193-1195), la tracizoline et la benazoline (Pigini, M,; Bousquet, P,; Carotti, A,; Dontenwill, M,; Giannella, M,; Moriconi, R,; Piergentili, A,; Quaglia, W,; Tayebati, S. K,; Brasili, L. Imidazoline Receptors: Qualitative Structure-Activity Relationships and Discovery of Tracizoline and Benazoline. Two Ligands with High Affinity and Unprecedented Selectivity. Bioorg. Med. Chem. 1997, 5, 833-841) ne modifient pas la pression artérielle ou la modifient peu.

Par ailleurs, il existe plusieurs composés sélectifs des RI₁ par rapport aux Rα₂A mais qui ne sont pas sélectifs par rapport aux RI₂, tels que S23757 (Anastasiadou, M,; Danoun, S,; Crane, L,; Baziard-Mouysset, G,; Payard, M,; Caignard, D,-H,; Rettori, M,-C,; Renard, P. Synthesis and Pharmacological Evaluation of Imidazoline Sites I1 and I2 Selective Ligands. Bioorg. Med. Chem. 2001, 9, 585-592) et PMS 952 (Ye, H. F,; Dive, G,; Dehareng, D,; Heymans, F,; Godfroid, J. J. Structure-Activity Relationship on Adrenoreceptors and Imidazoline-Preferring Binding Sites (I1,2-PBS). Part 1 : Weak Intramolecular H-bond and Conformational Flexibility in a New I1-PBS-Selective Imidazoline Analogue, trans1-(4'-5'-Dihydro-1'H-imidazol-2'-yl)méthyl-2-hydroxyindane (PMS 952). Bioorg. Med. Chem. 2000, 8, 1861-1869).

Récemment, des composés sélectifs des RI₁, LNP 911 et LNP 906, qui sont des aminopyrrolines contrairement aux autres composés décrits ci-dessus et présentent des affinités nanomolaires pour le récepteur RI₁, ont été développés respectivement en tant que radioligand et radioligand de photoaffinité (Greney, H,; Urosevic, D,; Schann, S,; Dupuy, L,; Bruban, V,; Ehrhardt, J,-D,; Bousquet, P,; Dontenwill, M. [125I]2-(2-Chloro-4-iodo-phénylamino)-5-méthyl-pyrroline (LNP911), a High-Affinity Radioligand Selective for I1 Imidazoline Receptors. Mol. Pharmaco/. 2002, 62, 181-191), mais ne possèdent pas d'effet hypotenseur. Le LNP 911 se comporte plus comme un antagoniste tandis que LNP 906 qui est un ligand photoactivable est inutilisable dans des expérimentations in vivo.

Par conséquent, il existe un besoin de développer des composés qui soient sélectifs des RI₁, de manière à éviter les effets secondaires, qui possèdent toujours une activité hypotensive et qui puissent être utilisés en monothérapie dans le syndrome métabolique.

Un des objets de la présente invention est de fournir des composés sélectifs des RI₁ n'interagissant donc pas ou très peu avec les Rα₂A et les RI₂. De plus, les composés sont des agonistes des RI₁.

Un autre objet de l'invention est de fournir des médicaments actifs dans le syndrome métabolique, utilisables en monothérapie et ne présentant pas les effets secondaires des médicaments actuels.

Un autre objet de l'invention est de fournir des compositions pharmaceutiques comprenant lesdits médicaments actifs.

Ainsi, la présente invention concerne en particulier des composés de formule générale (I) 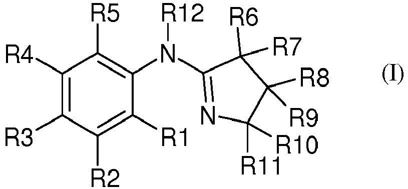 dans laquelle :
a) soit R12 représente H, et
   - R1 et R2 représentent indépendamment les uns des autres :
      un halogène, un alkyle en C1 à C8 linéaire ou ramifié, un alcène en C2-C8, un alcoxy en C1 à C8 linéaire ou ramifié, un cycloalkyle en C3-C6, un bicycloalkyle en C5-C6, une chaîne polyéther, un perfluoroalkyle en C1-C5, un acyle en C1-C8, OH, SH, une amine primaire, secondaire ou tertiaire, CN, CO₂H CO₂R' dans lequel R' est un alkyle linéaire ou ramifié en C1-C8, ou un cycloalkyle en C3-C6, ou
   - R1 et R2 forment ensemble un cycle en C5,
   - R3, R4 et R5 représentent indépendamment les uns des autres :
      un hydrogène, un halogène, un alkyle en C1 à C8 linéaire ou ramifié, un alcène en C2-C8, un alcoxy en C1 à C8 linéaire ou ramifié, un cycloalkyle en C3-C6, un bicycloalkyle en C5-C6, une chaîne polyéther, un perfluoroalkyle en C1-C5, un acyle en C1-C8, OH, SH, une amine primaire, secondaire ou tertiaire, CN, CO₂H, CO₂R' dans lequel R' est un alkyle linéaire ou ramifié en C1-C8, ou un cycloalkyle en C3-C6,
   - R6, R7, R9 et R11 représentent des hydrogènes,
   - R8 et R10 sont sélectionnés parmi le groupe consistant en un hydrogène et un alkyle en C1 à C5 linéaire ou ramifié, au moins l'un des deux étant un alkyle en C1 à C5 linéaire ou ramifié ;
b) soit R12 représente CH(R13)(CH₂) et forme un cycle en C5 avec R5, R13 représentant H ou CH₃,
   - R1, R2, R3 et R4 sont sélectionnés parmi le groupe consistant en un hydrogène, un halogène, un alkyle en C1 à C8 linéaire ou ramifié, un alcène en C2-C8, un alcoxy en C1 à C8 linéaire ou ramifié, un cycloalkyle en C3-C6, un bicycloalkyle en C5-C6, une chaîne polyéther, un perfluoroalkyle en C1-C5, un acyle en C1-C8, -OH, -SH, une amine primaire, secondaire ou tertiaire, -CN, -CO₂H, -CO₂R' dans lequel R' est un alkyle linéaire ou ramifié en C1-C8, et un cycloalkyle en C3-C6 ;
   - R6, R7, R9 et R11 représentent des hydrogènes,
   - R8 et R10 sont sélectionnés parmi le groupe consistant en un hydrogène et un alkyle en C1 à C5 linéaire ou ramifié, au moins l'un des deux étant un alkyle en C1 à C5 linéaire ou ramifié,
   à condition qu'au moins un parmi R1 et R13 ne soit pas un hydrogène,
   et leurs sels pharmacologiquement acceptables.

Les carbones portant R13 lorsque celui-ci est différent de H, ou R6 et R7 lorsqu'ils sont différents l'un de l'autre, ou R8 si il est différent de R9 et R10 si il est différent de R11, sont asymétriques et chacun desdits carbones peut donc être de configuration absolu (R) ou (S), ou (R,S).

Dans toute la description les expressions suivantes ont toujours la même signification:
- « alkyle en C-1 à C8 linéaire ou ramifié » désigne un méthyle, éthyle, n-propyle, n-butyle, n-pentyle, n-héxyle, n-heptyle ou n-octyle et tous leurs isomères, à savoir isopropyle, isobutyle, sec-butyl et tert-butyle, l'isopentane (ou 2-méthylbutyle) ou le néopentane (ou 2,2 diméthylpropyle), le 2,2-diméthylbutane, le 2,3-diméthylbutane, le 2-méthylpentane, le 3-méthylpentane, le 2-méthylhexane, le 3-méthylhexane, le 2,2-diméthylpentane, le 2,3-diméthylpentane, le 2,4-diméthylpentane, le 3,3-diméthylpentane, le 3-éthylpentane, le 2,2,3-triméthylbutane, le 2-méthylheptane, le 3-méthylheptane, le 4-méthylheptane, le 2,2-diméthylhexane, le 2,3-diméthylhexane, le 2,4-diméthylhexane, le 2,5-diméthylhexane, le 3,3-diméthylhexane, le 3,4-diméthylhexane, le 3-ethylhexane, le 2,2,3-triméthylpentane, le 2,2,4-triméthylpentane, le 2,3,3-triméthylpentane, le 2,3,4-triméthylpentane, le 2-méthyl-3-ethylpentane, le 3-méthyl-3-éthylpentane, le tetraméthylbutane.
   Ledit alkyle peut également être substitué, notamment par un alcool, un thiol, un éther, un halogène, un nitrile, une amine primaire, secondaire ou tertiaire;
- « alcène en C2-C8 » désigne un éthylène, propène, butène, pentène, héxène, heptène ou octène et tous leurs isomères.
   Ledit alcène peut également être substitué, notamment par un alcool, un thiol, un éther, un halogène, un nitrile, une amine primaire, secondaire ou tertiaire;
- un « acyle en C1-C8 » désigne un groupe -C(O)-alkyle en C1-C8 dont l'alkyle est tel que ci-dessus défini;
- un « sulfonylalkyle en C1-C8» désigne un groupe S(O)₂O-alkyle dans lequel l'alkyle en C1-C8 est tel que ci-dessus défini;
- un halogène désigne un brome, un chlore, un fluor ou un iode;
- un « alcoxy en C1-C8 linéaire ou ramifié » désigne un O-alkyle, c'est-à-dire un alkyle en C1-C8 linéaire ou ramifié ayant la même définition que ci-dessus, relié par un oxygène à la molécule de formule (I).
   Ledit alcoxy peut également être substitué, notamment par un alcool, un thiol, un éther, un halogène, un nitrile, une amine primaire, secondaire ou tertiaire;
- un « cycloalkyle en C3-C6 » désigne un cyclopropyle, cyclobutyle, cyclopentyle ou cyclohéxyle ;
- un « bicycloalkyle en C5-C6 » désigne deux cycles cycloalkyles en C5 et/ou C6 fusionnés;
   Lesdits cycloalkyles ou bicycloalkyles peuvent également être substitués, notamment par un alcool, un thiol, un éther, un halogène, un nitrile, une amine primaire, secondaire ou tertiaire;
- une chaîne polyéther désigne une chaîne O-(CH₂-CH₂-O)ₙCH₂-CH₂-OR", n variant de 0 à 9 et R" représentant un alkyle ou alcène ou cycloalkyle tels que définis ci-dessus ;
- un « perfluoroalkyle » en C1-C5 désigne un alkyle en C1-C5, tel que défini ci-dessus, dont tous les hydrogènes sont totalement substitués par un fluor; par exemple CF₃, C₂F₅, C₃F₇, C₄F₉, C₅F₁₁,
- une amine primaire, secondaire ou tertiaire désigne un groupe -NRₐR_{b} dans lequel Rₐ et R_{b} désignent indépendamment l'un de l'autre H, un alkyle linéaire ou ramifié en C1-C6, un cycloalkyle en C3-C6, un acyle en C1-C6.

Lorsque R12 représente CH(R13)(CH₂) et forme un cycle à 5C avec R5 qui représente CH₂, R13 représentant H ou CH₃, la formule I-1 suivante est obtenue :
- n = 0, cycle à 5C :

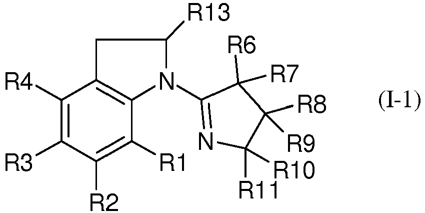

Dans un mode de réalisation préféré, les composés présentent la formule I-1.

L'expression « sels pharmacologiquement acceptables » signifie que les composés de la formule I, définie ci-dessus, lorsqu'ils possèdent un radical représentant une amine, peuvent exister sous forme d'ammonium par réaction d'un acide inorganique ou d'un acide organique sur l'amine.

Des exemples d'acides inorganiques permettant l'obtention de sels pharmacologiquement acceptables incluent sans être limités à ceux-ci l'acide chlorhydrique, l'acide bromhydrique, l'acide nitrique, l'acide carbonique, l'acide formique, l'acide monohydrogénocarbonique, l'acide phosphorique, l'acide monohydrogénophosphorique, l'acide dihydrogénophosphorique, l'acide perchlorique, l'acide sulfurique, l'acide monohydrogénosulfurique, l'acide iodhydrique.

Des exemples d'acides organiques permettant l'obtention de sels pharmacologiquement acceptables incluent sans être limités à ceux-ci l'acide acétique, l'acide lactique, l'acide propionique, l'acide butyrique, l'acide isobutyrique, l'acide palmique, l'acide maléique, l'acide glutamique, l'acide hydroxymaléique, l'acide malonique, l'acide benzoïque, l'acide succinique, l'acide glycolique, l'acide subérique, l'acide fumarique, l'acide mandélique, l'acide phthalique, l'acide salicylique, l'acide benzènesulfonique, l'acide *p*-toluènesulfonique, l'acide citrique, l'acide tartrique, l'acide méthanesulfonique, l'acide hydroxynaphthoïque.

Les sels d'acides aminés, tels que les arginates et leurs équivalents sont également inclus ainsi que les sels d'acides organiques tels que l'acide glucuronique ou l'acide galacturonique et leurs équivalents (voir, par exemple, Berge et al, "Pharmaceutical Salts", Journal de Pharmaceutical Science, 1977, 66, 1-19).

Les halogénures d'alkyle permettant l'obtention de sels pharmacologiquement acceptables incluent sans être limités à ceux-ci les bromure, iodure, fluorure ou chlorure d'alkyle dans lesquels ledit résidu alkyle est saturé ou non saturé, linéaire ou ramifié de 1 à 20 atomes de carbone, ou un groupe O-cycloalkyle de 3 à 8 atomes de carbone.

Lorsque les composés de formule I selon l'invention possèdent un radical représentant une acide ou un OH, notamment un phénol, ils peuvent exister sous forme de carboxylate, alcoolate ou phénate, par exemple de sodium, de potassium, de lithium ou d'ammonium par réaction de l'acide, alcool ou phénol avec une base telle que par exemple la soude, la potasse, l'hydroxyde de lithium, l'ammoniaque...

De façon inattendue les inventeurs ont observé que les composés de l'invention de par leur structure pyrroline originale sont sélectifs du récepteur aux imidazolines RI₁ par rapport au récepteur α₂-adrénergique, le rapport Ki (RAα₂)/Ki(RI₁) variant de 100 à plus de 10000.

Les composés de l'invention sont également sélectifs du récepteur aux imidazolines RI₁ par rapport au récepteur aux imidazolines RI₂, le rapport Ki (RI₂)/Ki(RI₁) étant environ égal à 1000.

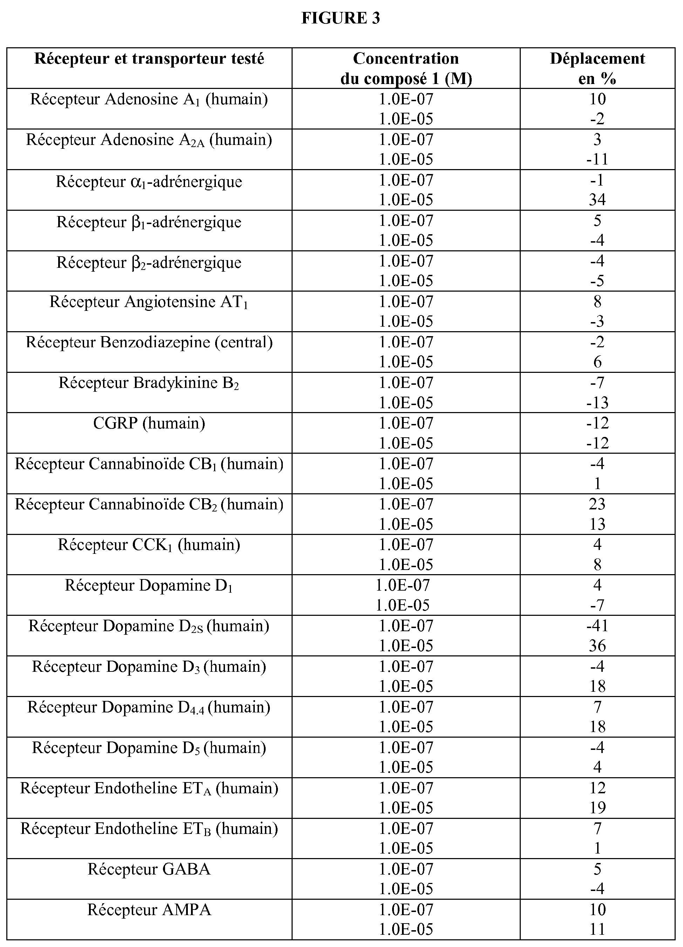

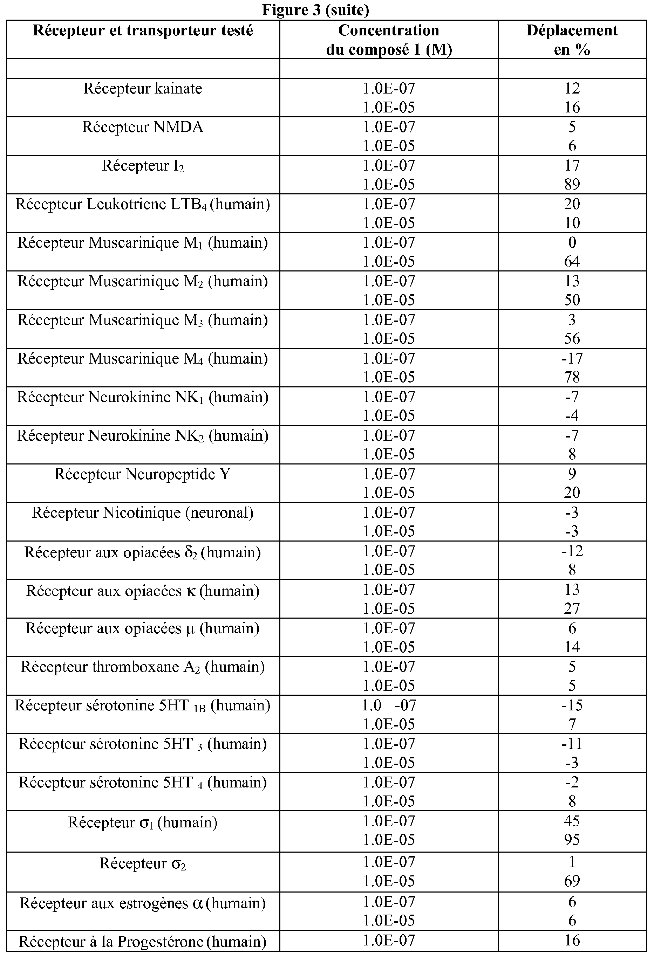

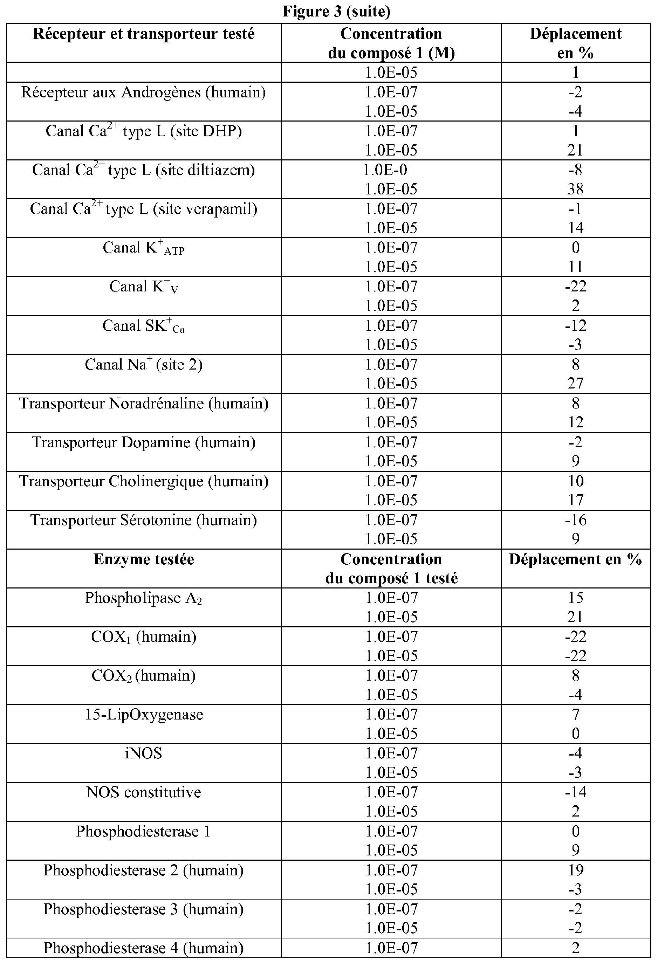

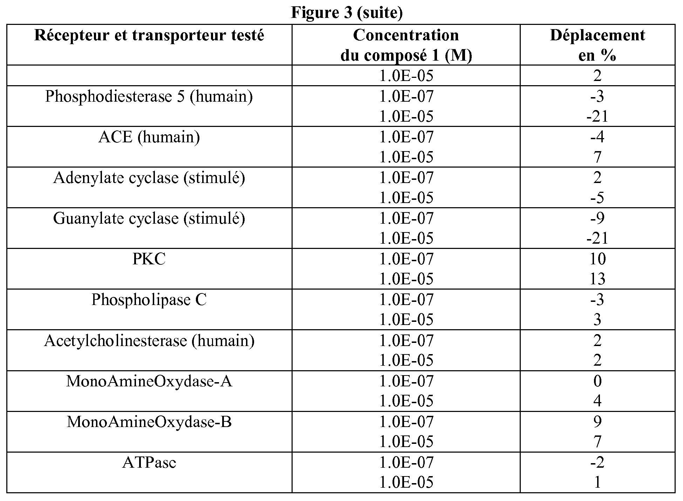

De plus, les composés de l'invention sont également sélectifs du récepteur aux imidazolines RI₁ par rapport à plus d'une cinquantaine d'autres cibles potentielles, les récepteurs ou enzymes, notamment ceux présentés dans le tableau de la figure 3.

Enfin, afin de présenter l'effet thérapeutique désiré, les composés de l'invention sont des agonistes du récepteur aux imidazolines RI₁. Cet effet agoniste peut être mesuré par une quelconque méthode disponible pour l'homme du métier. Notamment, il peut être testé en mesurant la capacité hypotensive des composés.

Dans un mode de réalisation avantageux, la présente invention concerne des composés tels que ci-dessus définis, de formule générale (I) dans laquelle :
- R1, R2, R3, R4 et R5 représentent indépendamment les uns des autres :
   H, un halogène, un alkyle en C1 à C8 linéaire ou ramifié, un alcène en C2-C8, un alcoxy en C1 à C8 linéaire ou ramifié, un cycloalkyle en C3-C6, un bicycloalkyle en C5-C6, une chaîne polyéther, un perfluoroalkyle en C1-C5, un acyle en C1-C8, OH, SH, une amine primaire, secondaire ou tertiaire, CN, CO₂H CO₂R' dans lequel R' est un alkyle linéaire ou ramifié en C1-C8, ou un cycloalkyle en C3-C6,
      R1 et R2 et/ou R2 et R3 et/ou R3 et R4 et/ou R4 et R5 pouvant également former ensemble un cycle en C4-C6,
- R6, R7, R8, R9, R10 et R11 représentent indépendamment les uns des autres:
   H, un alkyle en C1 à C8 linéaire ou ramifié, un alcène en C2-C8, un cycloalkyle en C3-C6,
- R12 représente H, un alkyle en C1 à C8 linéaire ou ramifié, un alcène en C2-C8, un acyle en C1-C8, un sulfonylalkyle en C1-C8.

Dans un mode de réalisation avantageux, la présente invention concerne des composés tels que ci-dessus définis, de formule générale (Ia) suivante : 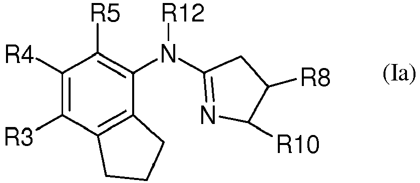 dans laquelle R8 et R10 représentent indépendamment l'un de l'autre H ou un alkyle C1 à C5 linéaire ou ramifié, en particulier CH₃, R3 à R5 sont tels que définis ci-dessus et R12 représente H, un alkyle en C1 à C6 linéaire ou ramifié, un alcène en C2-C8, un acyle en C1-C8, un sulfonylalkyle en C1-C8.

De préférence, R12 est un hydrogène, et R8 et R10 sont sélectionnés parmi le groupe consistant en un hydrogène et un alkyle en C1 à C5 linéaire ou ramifié, au moins l'un des deux étant un alkyle en C1 à C5 linéaire ou ramifié. Plus particulièrement, R8 et R10 sont sélectionnés parmi le groupe consistant en un hydrogène et un méthyle ou isobutyle de manière à ce que l'un d'eux soit un hydrogène et l'autre un méthyle ou isobutyle, de préférence un méthyle. Dans un mode de réalisation préféré, R3 est un hydrogène. Dans un mode de réalisation encore plus préféré, R3, R4 est R5 sont des hydrogènes.

Dans un autre mode de réalisation avantageux, la présente invention concerne des composés tels que ci-dessus définis, de formule générale (Ia) suivante : 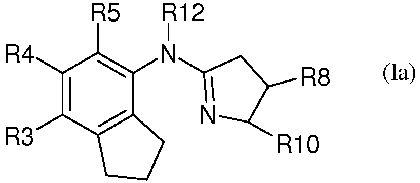 dans laquelle
R3 et R12 sont des hydrogènes,
R8 et R10 sont sélectionnés parmi le groupe consistant en un hydrogène et un alkyle en C1 à C5 linéaire ou ramifié, au moins l'un des deux étant un alkyle en C1 à C5 linéaire ou ramifié, et
R4 et R5 sont sélectionnés indépendamment parmi le groupe consistant en un hydrogène, un halogène, un alkyle en C1 à C3 linéaire ou ramifié, un alcoxy en C1 à C3 linéaire ou ramifié, un perfluoroalkyle en C1-C3, un acyle en C1-C3, OH, SH, une amine primaire, secondaire ou tertiaire, CN, CO₂H et CO₂R' dans lequel R' est un alkyle linéaire ou ramifié en C1-C3, de préférence parmi groupe consistant en un halogène, un alkyle en C1 à C3 linéaire ou ramifié, un alcoxy en C1 à C3 linéaire ou ramifié, un perfluoroalkyle en C1-C3, et un acyle en C1-C3.

De préférence, R4 et R5 sont des hydrogènes.

Dans un autre mode de réalisation avantageux, la présente invention concerne des composés tels que ci-dessus définis, de formule générale (Ib) suivante : 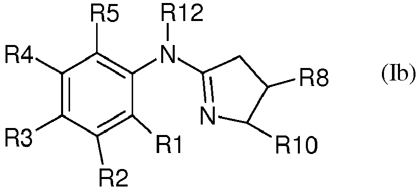 dans laquelle R8 et R10 représentent indépendamment les uns des autres H ou un alkyle C1 à C5 linéaire ou ramifié, en particulier CH₃, R1 et R2 représentent indépendamment les uns des autres H, CH₃ ou C1, R3 à R5 sont tels que définis ci-dessus et R12 représente H, un alkyle en C1 à C8 linéaire ou ramifié, un alcène en C2-C8, un acyle en C1-C8, un sulfonylalkyle en C1-C8.

De préférence, R12 est un hydrogène. De préférence, R8 et R10 sont sélectionnés parmi le groupe consistant en un hydrogène et un alkyle en C1 à C5 linéaire ou ramifié, au moins l'un des deux étant un alkyle en C1 à C5 linéaire ou ramifié. Plus particulièrement, R8 et R10 sont sélectionnés parmi le groupe consistant en un hydrogène et un méthyle ou isobutyle de manière à ce que l'un d'eux soit un hydrogène et l'autre un méthyle ou isobutyle, de préférence un méthyle. De préférence, R1 et R2 représentent indépendamment les uns des autres CH₃ ou C1. En particulier, R1 est un méthyle et R2 est un méthyle ou un chlorure. Dans un mode de réalisation encore plus préféré, R3, R4 est R5 sont des hydrogènes.

Dans un autre mode de réalisation avantageux, la présente invention concerne des composés tels que ci-dessus définis, de formule générale (Ib) suivante : 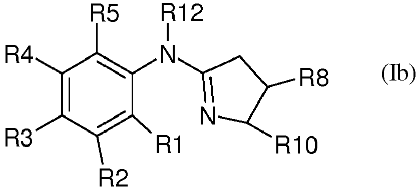 dans laquelle
R12 est un hydrogène,
R8 et R10 sont sélectionnés parmi le groupe consistant en un hydrogène et un alkyle en C1 à C5 linéaire ou ramifié, au moins l'un des deux étant un alkyle en C1 à C5 linéaire ou ramifié,
R1 et R2 sont sélectionnés indépendamment parmi le groupe consistant en un halogène, un alkyle en C1 à C3 linéaire ou ramifié, un alcoxy en C1 à C3 linéaire ou ramifié, un perfluoroalkyle en C1-C3, et un acyle en C1-C3, et
R3, R4 et R5 sont sélectionnés indépendamment parmi le groupe consistant en un hydrogène, un halogène, un alkyle en C1 à C3 linéaire ou ramifié, un alcoxy en C1 à C3 linéaire ou ramifié, un perfluoroalkyle en C1-C3, un acyle en C1-C3, OH, SH, une amine primaire, secondaire ou tertiaire, CN, CO₂H et CO₂R' dans lequel R' est un alkyle linéaire ou ramifié en C1-C3, de préférence parmi groupe consistant en un halogène, un alkyle en C1 à C3 linéaire ou ramifié, un alcoxy en C1 à C3 linéaire ou ramifié, un perfluoroalkyle en C1-C3, et un acyle en C1-C3.

De préférence, R1 et R2 sont sélectionnés indépendamment parmi le groupe consistant en un halogène et un alkyle en C1 à C3 linéaire ou ramifié. De manière encore plus préféré, R1 et R2 représentent indépendamment les uns des autres CH₃ ou C1.

De préférence, R3, R4 et R5 sont des hydrogènes.

Dans un autre mode de réalisation avantageux, la présente invention concerne des composés tels que ci-dessus définis, de formule (Ia-1) suivante: 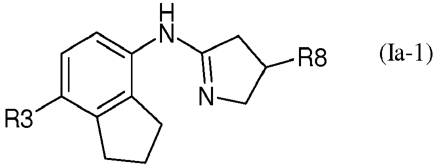 dans laquelle R3 et R8 sont tels que définis ci-dessus. De préférence, R8 est un alkyle en C1 à C5 linéaire ou ramifié, en particulier un méthyle ou un isobutyle, de préférence un méthyle. De préférence, R3 est un hydrogène.

Dans un autre mode de réalisation avantageux, la présente invention concerne des composés tels que ci-dessus définis, de formule (Ib-1) suivante: 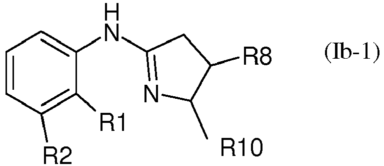 dans laquelle R1, R2, R8 et R10 sont tels que définis ci-dessus. De préférence, R8 et R10 sont sélectionnés parmi le groupe consistant en un hydrogène et un alkyle en C1 à C5 linéaire ou ramifié, au moins l'un des deux étant un alkyle en C1 à C5 linéaire ou ramifié. Plus particulièrement, R8 et R10 sont sélectionnés parmi le groupe consistant en un hydrogène et un méthyle ou isobutyle de manière à ce que l'un d'eux soit un hydrogène et l'autre un méthyle ou isobutyle, de préférence un méthyle. De préférence, R1 et R2 sont sélectionnés parmi le groupe consistant en un halogène, un alkyle en C1 à C8 linéaire ou ramifié, un alcoxy en C1 à C8 linéaire ou ramifié, un perfluoroalkyle en C1-C5, un acyle en C1-C8, OH, SH, une amine primaire, secondaire ou tertiaire, CN, CO₂H, et CO₂R' dans lequel R' est un alkyle linéaire ou ramifié en C1-C8, ou forment ensemble un cycle en C5. Plus particulièrement, R1 et R2 sont sélectionnés parmi le groupe consistant en un halogène, un alkyle en C1 à C3 linéaire ou ramifié, un alcoxy en C1 à C3 linéaire ou ramifié, un perfluoroalkyle en C1-C3, et un acyle en C1-C3, ou forment ensemble un cycle en C5. De préférence, R1 et R2 représentent indépendamment les uns des autres CH₃ ou C1. En particulier, R1 est un méthyle et R2 est un méthyle ou un chlorure.

Dans un autre mode de réalisation avantageux, la présente invention concerne des composés tels que ci-dessus définis, choisis parmi l'une des formules suivantes:

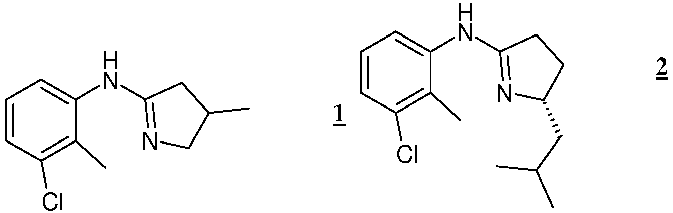

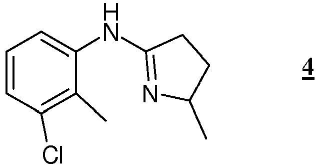

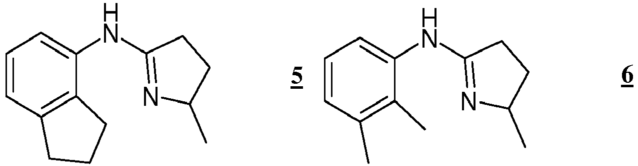

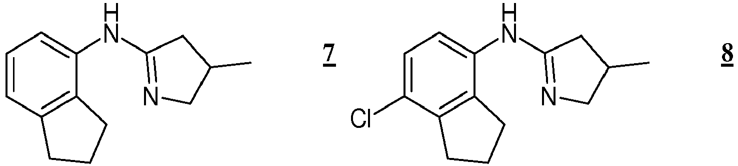

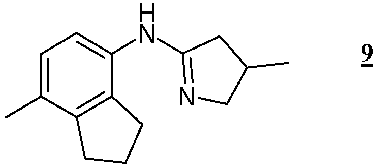

De préférence, les composés sont choisis parmi le groupe consistant en

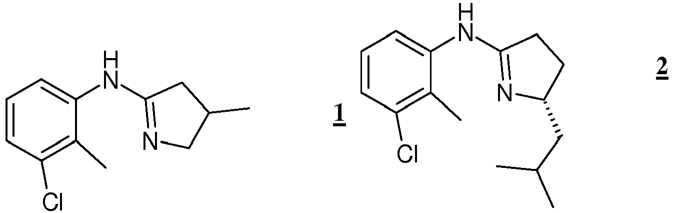

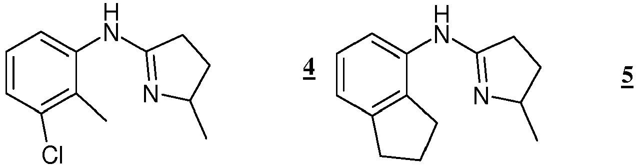

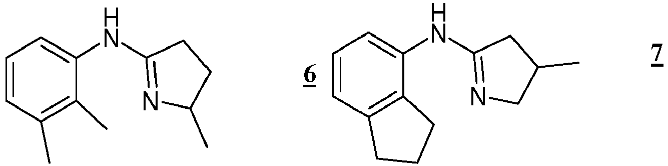

Dans un autre mode de réalisation avantageux, la présente invention concerne des composés tels que ci-dessus définis, de formule générale (Ic) suivante : 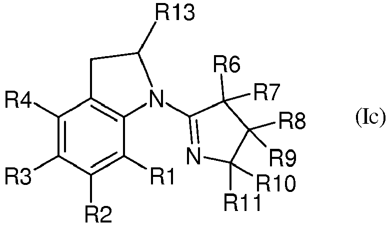 dans laquelle, R1 à R4 et R6 à R11 et sont tels que définis ci-dessus et R13 représente H ou CH₃.

Dans un mode de réalisation particulier des composés de formule générale (Ic), R13 représente un méthyle.

Dans un mode de réalisation particulier des composés de formule générale (Ic), R1 n'est pas un hydrogène.

Dans un mode de réalisation préféré des composés de formule générale (Ic), R13 est un méthyle et/ou R1 n'est pas un hydrogène. Ainsi, lorsque R13 est un hydrogène, alors R1 n'est pas un hydrogène. De même, au moins un parmi R1 et R13 n'est pas un hydrogène,

R1 est sélectionné parmi le groupe consistant en un hydrogène, un halogène, un alkyle en C1 à C8 linéaire ou ramifié, un alcène en C2-C8, un alcoxy en C1 à C8 linéaire ou ramifié, un cycloalkyle en C3-C6, un bicycloalkyle en C5-C6, une chaîne polyéther, un perfluoroalkyle en C1-C5, un acyle en C1-C8, OH, SH, une amine primaire, secondaire ou tertiaire, CN, CO₂H, CO₂R' dans lequel R' est un alkyle linéaire ou ramifié en C1-C8, et un cycloalkyle en C3-C6, de préférence parmi le groupe consistant en un hydrogène, un halogène, un alkyle en C1 à C8 linéaire ou ramifié, un alcène en C2-C8, un alcoxy en C1 à C8 linéaire ou ramifié, une chaîne polyéther, un perfluoroalkyle en C1-C5, un acyle en C1-C8, OH, SH, une amine primaire, secondaire ou tertiaire, CN, CO₂H et CO₂R' dans lequel R' est un alkyle linéaire ou ramifié en C1-C8, de manière encore plus préférée parmi le groupe consistant en un hydrogène, un halogène, un alkyle en C1 à C3 linéaire ou ramifié, un alcoxy en C1 à C3 linéaire ou ramifié, un perfluoroalkyle en C1-C3, et un acyle en C1-C3. Dans un mode de réalisation préféré, R1 est un hydrogène ou un alkyle en C1 à C3 linéaire ou ramifié, de préférence un méthyle. De préférence, R2, R3 et R4 sont des hydrogènes.

Dans un mode de réalisation particulier des composés de formule générale (Ic), parmi R1, R2, R3 et R4, trois d'entre eux sont des hydrogènes et le dernier est sélectionné parmi le groupe consistant en H, un halogène, un alkyle en C1 à C8 linéaire ou ramifié, un alcène en C2-C8, un alcoxy en C1 à C8 linéaire ou ramifié, un cycloalkyle en C3-C6, un bicycloalkyle en C5-C6, une chaîne polyéther, un perfluoroalkyle en C1-C5, un acyle en C1-C8, OH, SH, une amine primaire, secondaire ou tertiaire, CN, CO₂H CO₂R' dans lequel R' est un alkyle linéaire ou ramifié en C1-C8, et un cycloalkyle en C3-C6, de préférence parmi le groupe consistant en H, un halogène, un alkyle en C1 à C8 linéaire ou ramifié, un alcène en C2-C8, un alcoxy en C1 à C8 linéaire ou ramifié, une chaîne polyéther, un perfluoroalkyle en C1-C5, un acyle en C1-C8, OH, SH, une amine primaire, secondaire ou tertiaire, CN, CO₂H et CO₂R' dans lequel R' est un alkyle linéaire ou ramifié en C1-C8, de manière encore plus préférée parmi le groupe consistant en H, un halogène, un alkyle en C1 à C3 linéaire ou ramifié, un alcoxy en C1 à C3 linéaire ou ramifié, un perfluoroalkyle en C1-C3, et un acyle en C1-C3. Dans un mode de réalisation préféré, parmi R1, R2, R3 et R4, trois d'entre eux sont des hydrogènes et le dernier est un hydrogène ou un alkyle en C1 à C3 linéaire ou ramifié, de préférence un hydrogène ou un méthyle. De préférence, R2, R3 et R4 sont des hydrogènes.

Dans un mode de réalisation particulier des composés de formule générale (Ic), R6, R7, R9 et R11 sont des hydrogènes, et R8 et R10 sont choisis indépendamment parmi le groupe consistant en H, un alkyle en C1 à C8 linéaire ou ramifié, un alcène en C2-C8, un cycloalkyle en C3-C6, un perfluoroalkyle en C1-C5, de préférence parmi le groupe consistant en H et un alkyle en C1 à C8 linéaire ou ramifié, de manière encore plus préférée parmi le groupe consistant en H et un alkyle en C1 à C3 linéaire ou ramifié. De préférence, au moins un parmi R8 et R10 est un hydrogène. Dans un mode de réalisation préféré, R6, R7, R9 et R11 sont des hydrogènes, et R8 et R10 sont choisis parmi le groupe consistant en H et un alkyle en C1 à C3 linéaire ou ramifié, de préférence parmi H et un méthyle, au moins l'un des deux étant un hydrogène.

La présente invention concerne en particulier des composés tels que ci-dessus définis, de formule générale (Ic)
dans laquelle,
- R1, R2, R3 et R4 sont sélectionnés parmi le groupe consistant en un hydrogène, un halogène, un alkyle en C1 à C3 linéaire ou ramifié, un alcoxy en C1 à C3 linéaire ou ramifié, un perfluoroalkyle en C1-C3, un acyle en C1-C3, OH, SH, une amine primaire, secondaire ou tertiaire, -CN, -CO₂H, et -CO₂R' dans lequel R' est un alkyle linéaire ou ramifié en C1-C3;
- R8 et R10 sont sélectionnés parmi le groupe consistant en un hydrogène et un alkyle en C1 à C5 linéaire ou ramifié, au moins l'un des deux étant un hydrogène,
à condition qu'au moins un parmi R1 et R13 ne soit pas un hydrogène.

Dans un mode de réalisation particulier des composés de formule générale (Ic),
- R2, R3, R4, R6, R7, R9, R10 et R11 sont des hydrogènes;
- R13 est H et R1 est un halogène et un alkyle en C1 à C3 linéaire ou ramifié; ou
   R13 est un méthyle, et R1 est sélectionné parmi le groupe consistant en un hydrogène, un halogène et un alkyle en C1 à C3 linéaire ou ramifié ; et
- R8 et R10 sont sélectionnés parmi le groupe consistant en un hydrogène et un alkyle en C1 à C5 linéaire ou ramifié, au moins l'un des deux étant un hydrogène.

Dans un mode de réalisation encore plus particulier des composés de formule générale (Ic),
- R2, R3, R4, R6, R7, R9, R10 et R11 sont des hydrogènes;
- R13 est H et R1 est un méthyle; ou
   R13 est un méthyle, et R1 est sélectionné parmi le groupe consistant en un hydrogène et un méthyle ; et
- R8 et R10 sont sélectionnés parmi le groupe consistant en un hydrogène et un méthyle, au moins l'un des deux étant un hydrogène.

Dans un autre mode de réalisation avantageux, la présente invention concerne des composés tels que ci-dessus définis, de formule (Ic-1) suivante: 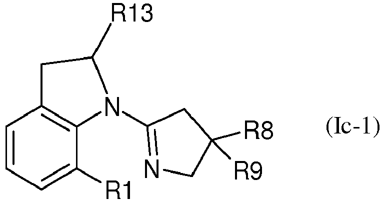 dans laquelle, R1, R8 et R9 sont tels que définis ci-dessus et R13 représente H ou CH₃.

De préférence, R9 est un hydrogène et au moins un groupe parmi R1, R8 et R13 n'est pas un hydrogène. Dans un mode de réalisation, deux groupes parmi R1, R8 et R13 ne sont pas des hydrogènes.

De préférence, R1 est choisi parmi le groupe consistant en H, un halogène, un alkyle en C1 à C8 linéaire ou ramifié, un alcène en C2-C8, un alcoxy en C1 à C8 linéaire ou ramifié, une chaîne polyéther, un perfluoroalkyle en C1-C5, un acyle en C1-C8, OH, SH, une amine primaire, secondaire ou tertiaire, CN, CO₂H, et CO₂R' dans lequel R' est un alkyle linéaire ou ramifié en C1-C8, de manière encore plus préférée parmi le groupe consistant en H, un halogène, un alkyle en C1 à C3 linéaire ou ramifié, un alcoxy en C1 à C3 linéaire ou ramifié, un perfluoroalkyle en C1-C3, et un acyle en C1-C3. En particulier, R1 est choisi parmi le groupe consistant en H, un halogène, un alkyle en C1 à C3 linéaire ou ramifié. En particulier, R1 est H ou un méthyle.

De préférence, R8 est choisi parmi le groupe consistant en H et un alkyle en C1 à C8 linéaire ou ramifié, de manière encore plus préférée parmi le groupe consistant en H et un alkyle en C1 à C3 linéaire ou ramifié. En particulier, R8 est H ou un méthyle.

Dans un mode de réalisation particulier des composés de formule générale (Ic-1),
- R9 est un hydrogène ;
- R13 est H et R1 est un halogène et un alkyle en C1 à C3 linéaire ou ramifié; ou
   R13 est un méthyle et R1 est sélectionné parmi le groupe consistant en un hydrogène, un halogène et un alkyle en C1 à C3 linéaire ou ramifié ; et
- R8 et R10 sont sélectionnés parmi le groupe consistant en un hydrogène et un alkyle en C1 à C5 linéaire ou ramifié, au moins l'un des deux étant un hydrogène.

Dans un mode de réalisation encore plus particulier des composés de formule générale (Ic-1),
- R9 est un hydrogène;
- R13 est H et R1 est un méthyle; ou
   R13 est un méthyle, et R1 est sélectionné parmi le groupe consistant en un hydrogène et un méthyle ; et
- R8 et R10 sont sélectionnés parmi le groupe consistant en un hydrogène et un méthyle, au moins l'un des deux étant un hydrogène.

Dans un autre mode de réalisation avantageux, la présente invention concerne des composés tels que ci-dessus définis, choisis parmi l'une des formules suivantes:

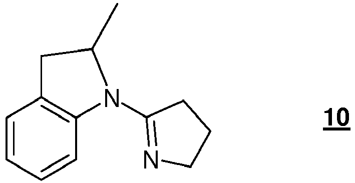

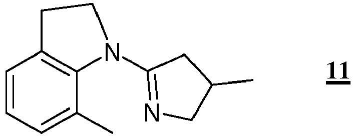

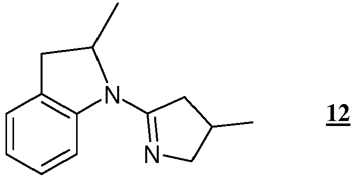

Les composés selon l'invention peuvent être synthétisés par des méthodes connues de l'homme du métier, décrits dans la littérature à partir des composés disponibles dans le commerce ou préparés selon des techniques connues de l'homme du métier.

Selon un autre aspect, la présente invention concerne des composés tels que définis ci-dessus, pour leur utilisation pour la prévention et/ou le traitement du syndrome métabolique.

Les composés de l'invention, sélectifs du récepteur RI₁, aux imidazolines permettent non seulement de disposer de composés dénués d'effets secondaires notamment dus à l'interaction avec le récepteur α₂A tel que la sédation, qui conservent un effet hypotenseur mais encore permettent de traiter, en monothérapie toutes les composantes du syndrome métabolique, à savoir l'hypertension artérielle, l'hypercholestérolémie, la résistance à l'insuline, l'intolérance au glucose et l'obésité abdominale, évitant ainsi l'emploi d'une association de 3 à 6 médicaments génératrice d'effets secondaires multiples et de surcoûts pour les systèmes d'assurance maladie.

La monothérapie et l'absence d'effets secondaires tels que la sédation observée chez l'animal devrait permettre l'utilisation des composés de l'invention dans le cadre de la prévention du syndrome métabolique ou de l'une ou plusieurs des composantes du syndrome métabolique chez des patients à risque pour l'une ou l'autre des dites composantes.

Selon un autre aspect, la présente invention concerne une composition pharmaceutique comprenant comme principe actif au moins un composé défini ci-dessus, en association avec un véhicule pharmaceutiquement acceptable.

Par véhicule pharmaceutiquement acceptable, il faut comprendre toute substance autre que le principe actif dans un médicament. Son addition est destinée à conférer des caractéristiques physico-chimiques et/ou biochimiques pour favoriser l'administration par voie orale, sublinguale, respiratoire, rectale, nasale, intestinale, parentérale, par injection intraveineuse, intrapéritonéale, intramusculaire, sous-cutanée, ou d'autres caractéristiques de consistance ou gustatives particulières, au produit final, en évitant de préférence les interactions, chimiques covalentes avec les principes actifs.

Les compositions pharmaceutiques de l'invention peuvent être sous forme de comprimés simples ou dragéifiés, de comprimés sublinguaux, de gélules, de glossettes, de capsules, de tablettes, de préparations injectables, d'aérosols, de gouttes nasales, de suppositoires, de crèmes, pommades ou gels dermiques.

Selon un autre aspect, la présente invention concerne une méthode de traitement du syndrome métabolique par administration chez un patient, notamment par voie orale, d'une composition pharmaceutique comprenant comme principe actif au moins un composé défini ci-dessus, en association avec un véhicule pharmaceutiquement acceptable, à une dose efficace.

Dans un mode de réalisation avantageux, la présente invention concerne une composition pharmaceutique telle que définie ci-dessus, administrable par voie orale.

Dans un mode de réalisation avantageux, le principe actif de la composition pharmaceutique administrable par voie orale définie ci-dessus, est à une dose comprise de 1 mg/kg à 100 mg/kg chez l'homme.

En deçà de 1 mg/kg, la dose est trop faible pour obtenir une activité dans le traitement du syndrome métabolique, au-delà de 100 mg/kg, il existe un risque d'apparition d'effets secondaires.

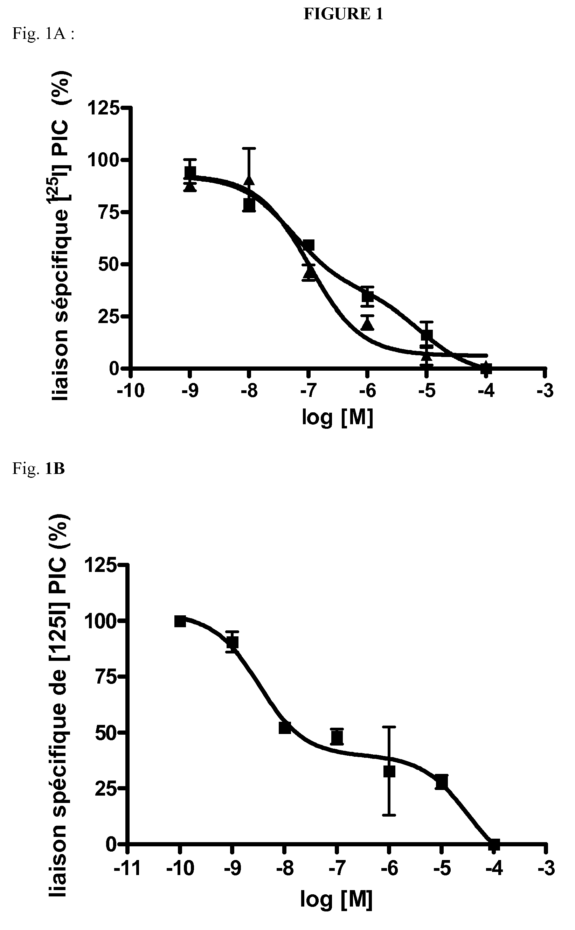

Les exemples A et 1 à 5 et les figures 1 à 8 qui suivent illustrent l'invention.

### PARTIE CHIMIQUE

Abréviations utilisées :
CDCl₃: Chloroforme deutérié
iPrOH: Isopropanol
Et₂O: Ether diéthylique
POCl₃: Trichlorure de phosphore
TEA: Triéthylamine
TMG: Tétraméthylguanidine

Tous les solvants ont été purifiés selon les procédures standard avant utilisation. Les chromatographies sur couche mince ont été effectuées sur plaque de silice 60F254 (Merck) et les taches ont été visualisées en utilisant une lampe UV. Les chromatographies flash ont été effectuées sur du gel de silice SI 60 (40-63µm) comme phase stationnaire. Les points de fusion (p,f,) ont été déterminés dans des capillaires ouverts avec un appareil Gallenkamp et sont non corrigés. Les spectres RMN ont été enregistrés dans CDCl₃ ou D₂O sur un spectromètre Brucker AV300. Les déplacements chimiques (δ) sont exprimés en parties par million (ppm), les constantes de couplages sont exprimées en Hertz (Hz). Les abréviations utilisées pour les multiplicités sont les suivantes : m : multiplet non spécifié, s : singulet, d : doublet, t : triplet, q : quadruplet, qn : quintuplet, hex : hexuplet, h : heptuplet. Les analyses élémentaires ont été effectuées au service de microanalyse de l'Université Louis Pasteur, Strasbourg, France. Les résultats analytiques obtenus pour C, H et N sont indiqués avec ± 0,4% des valeurs théoriques calculées. Tous les composés cibles ont été testés sous forme de chlorhydrate. Les chlorhydrates ont été préparés par addition d'une solution éthanolique de base (1 eq). Les chlorhydrates ont été recristallisés dans iPrOH-Et₂O.

### EXEMPLE A : Procédure générale pour la préparation des aminopyrrolines 1-27 de l'invention

Les composés sont synthétisés par réactions du lactame approprié avec l'aniline correspondante en présence de POCl₃ :

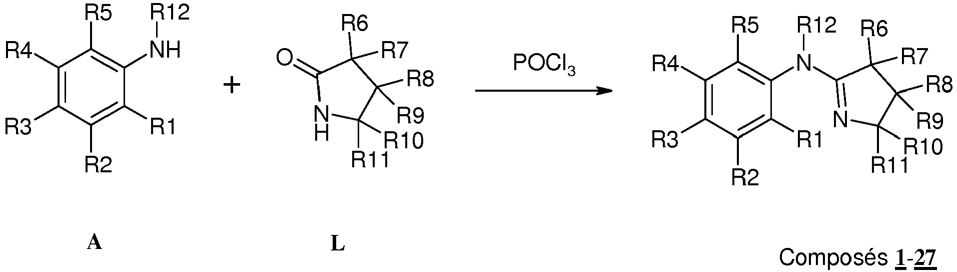

### A.1 : Procédure générale pour la synthèse des lactames L (28-33):

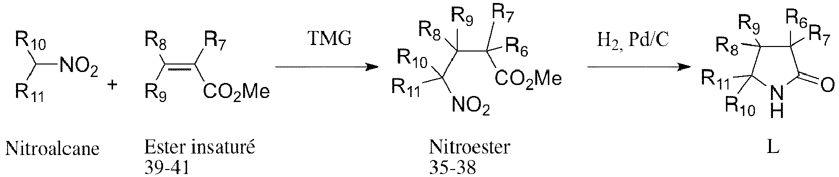

### A.1.1 : Préparation des nitroesters 35-38 : procédure générale

L'ester α,β-insaturé **(39-41)** (1 eq,) a été ajouté au nitroalkane en excès (5 eq,) sous atmosphère d'argon. Une quantité catalytique de tétraméthylguanidine (0,1 eq,) a été ajoutée au mélange qui a été agité pendant 12 h température ambiante. Le nitroalkane en excès a été distillé sous pression réduite et le résidu a été traité par HCl 2 N.

Le mélange a été extrait pat Et₂O. La phase organique a été lavée à l'eau, puis par de la saumure et finalement séchée sur Na₂SO₄ anhydre avant que le solvant ne soit évaporé. Le produit a été distillé sous pression réduite pour donner de nitroester pur.

Méthyl **-4-nitro-butyrate** (**34**) produit commercial

Méthyl 3-Méthyl-4-nitro-butyrate (**35**). Rdt 85%, huile incolore, bp 69-70 °C (3 mmHg); ¹H NMR (CDCl₃) *δ*4,47 (dd, 1H, 1/2-CH₂, J= 6,3, J= 12,6); 4,34 (dd, 1H, 1/2-CH₂, J= 6,3, J= 12,6); 3,69 (s, 3H, CH₃); 2,78 (qd, 1H, CH, J= 6,8, J= 13,5); 2,46 (dd, 1H, 1/2-CH₂, J= 6,7, J= 16,2); 2,35 (dd, 1H, 1/2-CH₂, J= 6,8, J= 16,2); 1,09 (d, 3H, CH₃, J= 6,8).

Méthyl 4-Méthyl-4-nitro-pentanoate (**36**). Rdt 55%, huile incolore, bp 71-72 °C (2,5 mmHg); ¹H NMR (CDCl₃) *δ* 3,68 (s, 3H, OCH₃); 2,37-2,23 (m, 4H, 2xCH₂); 1,59 (s, 6H, 2xCH₃).

Méthyl 3,3-Diméthyl-4-nitro-butyrate **(37)**. Rdt 48%, huile incolore, bp 70-71 °C (1,5 mmHg) ; ¹H NMR (CDCl₃) *δ*4,52 (s, 2H, CH₂); 3,67 (s, 3H, CH₃); 2,45 (s, 2H, CH₂); 1,15 (s, 6H, 2xCH₃).

Méthyl 4-(2-méthyl-propyl)-4-nitro-pentanoate **(38)**, Rdt 45%, huile
¹H NMR (CDCl₃) *δ*3,75 (m, 1 H); 3,69 (s, 3 H); 2,39-2,24 (m, 4 H); 1,85 (m, 2 H); 1,60 (m, 1 H); 0,95 (d, J = 7 Hz, 6 H).

### A.1.2 Préparation des lactames L 30-33:

Le nitroester **34-38** (100 mmol) a été dissous dans 250 ml d'acide acétique glacial et 500 mg de Pd-C 10% a été ajouté. Le mélange a été hydrogéné à température ambiante et pression atmosphérique pendant 12h. Le mélange a été filtré et le solvant évaporé. Le produit a été ensuite dissous dans 100 ml d'éthanol absolu et alcalinisé par de la triéthylamine (TEA). Le mélange a été chauffé au reflux pendant 12h. Les solvants ont été évaporés sous pression réduite; le résidu a été dilué dans Et₂O et HCl 1N a été ajouté. La phase aqueuse a été extraite deux fois par Et2O.

Les phases organiques ont été séchées sur Na₂SO₄ et le solvant a été évaporé. Le produit a été purifié par distillation sous pression réduite pour conduire aux lactames **30-33.**

**Pyrrolidin-2-one** (**28**) produit commercial

**3-Méthyl-pyrrolidin-2-one** (**29**) produit commercial

**4-Méthyl-pyrrolidin-2-one** (**30**). Rdt 83%, huile incolore, (cristallise par la suite), bp 103 °C (6 mmHg) ; ¹H NMR (CDCl₃) *δ* 6,75 (br s, 1H, NH); 3,46 (dd, 1H, 1/2-CH₂, J= 7,6, J= 9,3); 2,93 (dd, 1H, 1/2-CH₂, J= 6,0, J= 9,5); 2,53-2,44 (m, 1H, CH); 2,46 (dd, 1H, 1/2-CH₂, J= 6,9, J= 16,3); 1,94 (dd, 1H, 1/2-CH₂, J= 7,0, J= 16,2); 1,10 (d, 3H, CH₃, J= 6,9).

**5,5-Diméthyl-pyrrolidin-2-one** (**31**)**.** Rdt 61%, huile incolore, (cristallise par la suite), bp 85-88 °C (2 mmHg) ; ¹H NMR (CDCl₃ *δ* 6,52 (br s, 1H, NH); 2,42 (t, 2H, CH₂, J= 8,0); 1,92 (t, 2H, CH₂, J= 8,0); 1,28 (s, 6H, 2xCH₃).

**4,4-Diméthyl-pyrrolidin-2-one** (**32**)**.** Rdt 73%, huile incolore, (cristallise par la suite), bp 94-95 °C (2 mmHg) ; ¹H NMR (CDCl₃) *δ* 6,68 (br s, 1H, NH); 3,06 (s, 2H, CH₂); 2,11 (s, 2H, CH₂); 1,16 (s, 6H, 2xCH₃).

**5-(2-méthyl-propyl)-pyrrolidin-2-one** (**33**), **Rdt 67%,** huile visqueuse.
¹H NMR (CDCl₃ *δ* 6,68 (m, 1 H); 3,75-3,70 (m, 1 H); 2,38-2,19 (m, 3 H); 1,72-1,60 (m, 2H); 1,68 (m, 1 H), 1,52-1,42 (m, 1 H); 1,36-1.26 (m, 1 H) 0,93 (d, J = 8 Hz, 6 H).

### A.1.3 Préparation des dérivés indanes 42-44

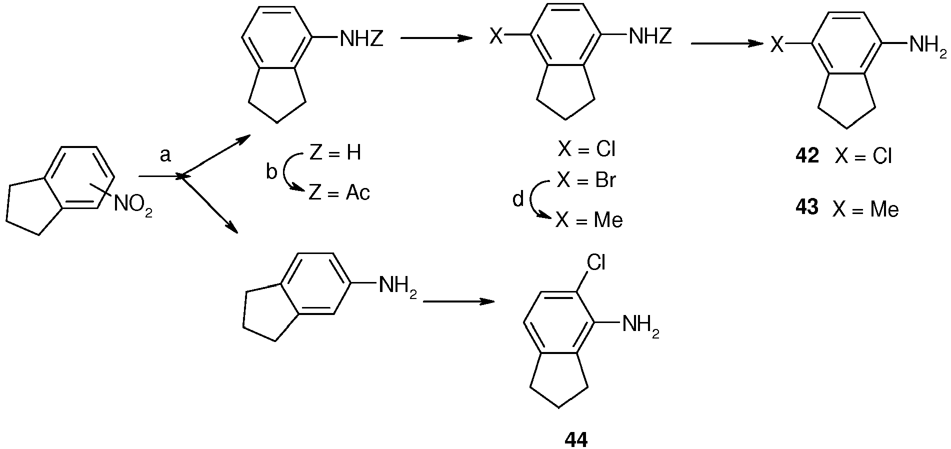

### a) Indan-4-ylamine et indan-5-ylamine

Le nitroindane (mélange commercial des isomères 4 et 5) a été soumis à une hydrogénation dans le méthanol avec du Pd/C 10 % à température ambiante pendant 12h. Les deux isomères d'indanylamine ont été séparés par chromatographie flash (AcOEt-hexane, 3-7) pour fournir l'indan-4-ylamine (huile brune qui cristallise) avec 53% de rendement et l'indan-5-ylamine (huile brune qui cristallise) avec 40% de rendement.

¹H NMR (CDCl₃) (Indan-4-ylamine) *δ* 7,69 (d, 1H, Har, J= 8,1); 7,18 (t, 1H, Har, J= 8,1); 7,04 (d, 1H, Har, J= 8,0); 6,88 (br s, 2H, NH₂); 2,93 (t, 2H, CH₂, J= 7,5); 2,84 (t, 2H, CH₂, J= 7,4); 2,11 (qn, 2H, CH₂, J= 7,5)

### b) Préparation du dérivé indane 42

L'indan-4-ylamine a été dissoute dans de l'anhydride acétique pur à 0°C. Le précipité résultant qui apparaît rapidement a été filtré et lavé avec de l'eau. Le produit a été récupéré avec un rendement de 90% (solide blanc gris) et est suffisamment pur pour être utilisé pour l'étape suivante sans autre purification.

¹H NMR (CDCl₃) *δ*7,73 (d, 1H, Har, J= 8,1); 7,15 (t, 1H, Har, J= 8,1); 7,02 (d, 1H, Har, J= 8,0); 6,96 (br s, 1H, NH); 2,95 (t, 2H, CH₂, J= 7,5); 2,81 (t, 2H, CH₂, J= 7,4); 2,18 (s, 3H, CH₃); 2,10 (qn, 2H, CH₂, J= 7,5).

Le produit obtenu ci-dessus (1g) a été dissous dans 20 ml d'acide acétique glacial et une solution de chlore dans l'acide acétique glacial, fraîchement préparée, a été ajoutée (1 eq). Après 20 min, 30 ml d'eau ont été ajoutés et le mélange a été agité pendant 10 min. Le précipité qui apparaît a été filtré et lavé avec une solution aqueuse saturée de carbonate de sodium, avec Na₂S₂O₅ 20% et avec de l'eau. Le produit a été séché au dessiccateur pour conduire au composé 7-chloro-4-acétamidoindane avec un rendement de 96% sous forme de solide blanc.

¹H NMR (CDCl₃) *δ* 7,72 (d, 1H, Har, J= 8,6); 7,12 (d, 1H, Har J= 8,6); 7,0 (br s, 1H, NH); 2,99 (t, 2H, CH₂, J= 7,5); 2,87 (t, 2H, CH₂, J= 7,5); 2,16 (s, 3H, CH₃); 2,11 (qn, 2H, CH₂, J= 7,5).

Le 7-chloro-4-acétamidoindane (1g) a été resuspendu dans 50 ml d'HCl 4N et le mélange a été chauffé au reflux pendant 2h. Après refroidissement, le mélange a été lavé avec de l'AcOEt, alcalinisé avec des pastilles de NaOH jusqu'à ce que le pH soit légèrement basique. Le mélange a été extrait avec Et₂O La phase organique a été lavée avec de l'eau, de la saumure et séchée sur sulfate de sodium anhydre. Le solvant a été évaporé et l'aniline **42** a été obtenue avec un rendement presque quantitatif.

¹H NMR (CDCl₃) *δ* 7,01 (d, 1H, Har, J= 8,4); 6,47 (d, 1H, Har, J= 8,4); 3,55 (br s, 2H, NH₂); 2,94 (t, 2H, CH₂, J=7,5); 2,83 (t, 2H, CH₂, J= 7,5); 2,13 (qn, 2H, CH₂, J= 7,4).

### c) Préparation du dérivé indane 43

Le mode opératoire est le même que pour l'aniline **42** sauf que du brome est utilisé à la place du chlore.

Le composé 7-bromo-4-acétamidoindane a été obtenu avec un rendement de 92% sous forme de solide blanc.

¹H NMR (CDCl₃) *δ* 7,65 (d, 1H, Har, J= 8,6); 7,28 (d, 1H, Har, J= 8,6); 6,89 (br s, 1H, NH); 2,97 (t, 2H, CH₂, J= 7,6); 2,90 (t, 2H, CH₂, J= 7,5); 2,18 (s, 3H, CH₃); 2,13 (qn, 2H, CH₂, J= 7,6). Le composé 7-bromo-4-acétamidoindane a été dissous dans le dioxane (10 ml) et le mélange a été dégazé et placé sous atmosphère d'argon. Pd(PPh₃)₄ (10% en moles) et 5 ml de Na₂CO₃ 2 N dégazé ont été ajoutés. Le triméthylboroxine (1,5 eq) a été ensuite ajouté au mélange par une seringue et le tout a été chauffé au reflux pendant 10h. Après refroidissement, le mélange réactionnel a été dilué avec 5 ml d'eau et filtré. Le mélange a été extrait avec du dichlorométhane, la phase organique a été séchée sur sulfate de sodium anhydre et le solvant a été évaporé sous pression réduite. Le produit a été purifié par chromatographie flash (AcOEt-heaxane ; 3-7) pour conduire au composé 7-méthyl-4-acétamidoindane avec un rendement de 80% sous forme de solide blanc.

¹H NMR (CDCl₃) *δ* 7,57 (d, 1H, Har, J= 8,2); 6,96 (d, 1H, Har, J= 8); 6,89 (br s, 1H, NH); 2,86 (t, 2H, CH₂, J= 7,4); 2,82 (t, 2H, CH₂, J= 7,4); 2,22 (s, 3H, CH₃(ar)); 2,17 (s, 3H, CH₃); 2,10 (qn, 2H, CH₂, J= 7,5).

Le 7-méthyl-4-acétamidoindane a été hydrolysé de la même manière que le 7-chloro-4-acétamidoindane ci-dessus. Le composé **43** a été obtenu sous forme d'huile brune avec un rendement de 95%.

¹H NMR (CDCl₃) *δ* 6,81 (d, 1H, Har, J= 7,7); 6,46 (d, 1H, Har, J= 7,8); 3,44 (br s, 2H, NH₂); 2,84 (t, 2H, CH₂, J= 7,5); 2,76 (t, 2H, CH₂, J= 7,4); 2,18 (s, 3H, CH₃(ar)); 2,12 (qn, 2H, CH₂, J= 7,5).

### d) Préparation du dérivé 44

Le protocole utilisé est le même que pour le dérivé **42** mais en partant de l'indan-5-ylamine.

¹H NMR (CDCl₃) *δ* 7,11 (s, 1H, Har); 6,72 (s, 1H, Har); 3,92 (br s, 2H, NH₂); 2,81 (t, 4H, 2×CH₂, J= 7,5); 2,03 (qn, 2H, CH2, J= 7,4).

### A.1.4 Préparation des aminopyrrolines 1-27

Une solution du lactame **28-33** approprié (5 mmol) et du dérivé aniline choisi a été préparée dans le dichloroéthane (10 ml) sous atmosphère d'argon. POCl₃ (1 eq) a été ajouté goutte à goutte au mélange, qui a été ensuite chauffé à 60°C pendant 6h. Le mélange a été refroidi et hydrolysé avec 5 ml d'une solution aqueuse saturée de Na₂CO₃. La phase aqueuse a été extraite deux fois avec du dichlorométhane. La phase organique a été séchée sur du sulfate de sodium anhydre et le solvant a été évaporé sous pression réduite. Le produit a été purifié par chromatographie flash (3% de TEA dans AcOEt) pour conduire à l'une des aminopyrrolines **1-27.**

Les produits obtenus sont caractérisés ci-dessous par leur spectre RMN.

### Chlorhydrate de (3-Chloro-2-méthyl-phényl)-(4-méthyl-4,5-dihydro-3H-pyrrol-2-yl)-amine (1).

¹H NMR (D₂O) *δ*7,45 (d, 1H, Har, J= 7,9); 7,25 (d, 1H, Har, J= 7,9); 7,20 (t, 1H, Har, J= 7,8); 3,73-3,68 (m, 1H, 1/2 CH₂); 3,22-3,16 (m, 2H, 1/2 CH₂ + CH); 2,77-2,71 (m, 2H, CH₂); 2,22 (s, 3H, CH₃); 1,10 (d, 3H, CH₃, J= 6,5). ¹³C NMR (D₂O) *δ*169,1; 135,5; 133,4; 132,3; 130,1; 128,1; 125,3; 54,2; 37,9; 29,5; 18,1; 14,2. Anal. (C₁₂H₁₅ClN₂,HCl) C, H, N.

### Chlorhydrate de (3-Chloro-2-méthyl-phényl)-(5-(2-méthyl-propyl)-4,5-dihydro-3H-pyrrol-2-yl)-amine (2).

¹H-NMR (400 MHz, MeOH-d4) δ 7,54 (d, 1 H, J = 8 Hz), 7,36 (m, 2 H), 4,12 (m, 1 H), 3,18 (m, 2 H), 2,36 (s, 3 H), 1,95 (s, 1 H), 1,66 (m, 2 H), 1,46 (m, 1 H), 0, 95 (d, 6 H, J = 8 Hz). ¹³C-NMR δ (100 MHz, MeOH-d4) δ 131,2, 129,4, 126,5, 61,6, 61,4, 31,5, 31,4, 27,9, 27,8, 26,2, 23,6, 23,5, 22,6, 22,4, 15,2
MS-ESI (m/z): [M+H]⁺ calc pour C₁₅H₂₂ClN₂ : 265. Trouvé: 265

### Chlorhydrate de (2,6-Diméthyl-phényl)-(5-méthyl-4,5-dihydro-3H-pyrrol-2-yl)-amine (3). (non compris dans l'invention)

¹H NMR (D₂O) *δ* 7,24 (d, 1H, Har, J= 7,6); 7,17 (t, 1H, Har, J= 7,7); 7,06 (d, 1H, Har, J= 7,6); 4,02 (h, 1H, CH, J= 6,4); 3,09-2,97 (m, 2H, CH₂); 2,37-2,31 (m, 1H, 1/2 CH₂); 2,24 (s, 3H, CH₃); 2,08 (s, 3H, CH₃); 1,86-1,75 (m, 1H, 1/2 CH₂); 1,13 (d, 3H, CH₃, J= 6,4). ¹³C NMR (D₂O) *δ* 168,2; 139,2; 133,5; 133,1; 131,2; 126,6; 124,0; 56,2; 43,8; 37,0; 28,6; 24,9; 17,2. Anal. (C₁₃H₁₈N₂,HCl) C, H, N.

### Chlorhydrate de (3-Chloro-2-méthyl-phényl)-(5-méthyl-4,5-dihydro-3H-pyrrol-2-yl)-amine (4).

¹H NMR (CDCl₃) *δ* 7,41 (d, 1H, Har, J= 7,8); 7,23 (d, 1H, Har, J= 7,9); 7,18 (t, 1H, Har, J= 7,8); 4,02 (h, 1H, CH, J= 6,4); 3,11-3,05 (m, 2H, CH₂); 2,38-2,30 (m, 1H, 1/2 CH₂); 2,25 (s, 3H, CH₃); 2,11 (s, 3H, CH₃); 1,88-1,78 (m, 1H, 1/2 CH₂); 1,13 (d, 3H, CH₃, J= 6,4). ¹³C NMR (D₂O) *δ* 168,7; 139,5; 133,4; 132,8; 130,5; 126,8; 123,7; 56,2; 32,3; 30,5; 29,7; 24,9; 20,2. Anal. (C₁₂H₁₅ClN₂, HCl) C, H, N.

### Chlorhydrate de Indan-4-yl-(5-méthyl-4,5-dihydro-3H-pyrrol-2-yl)-amine (5).

¹H NMR (D₂O *δ* 7,28 (d, 1H, Har, J= 7,5); 7,21 (t, 1H, Har, J= 7,6); 7,02 (d, 1H, Har, J= 7,6); 4,07 (h, 1H, CH, J= 6,4); 3,04-2,88 (m, 2H, CH₂); 2,88 (t, 2H, CH₂, J= 7,5); 2,74 (t, 2H, CH₂, J= 7,4); 2,37 (m, 1H, 1/2 CH₂); 2,00 (qn, 2H, CH₂, J= 7,3); 1,83-1,75 (m, 1H, 1/2 CH₂); 1,17 (d, 3H, CH₃, J= 6,4). ¹³C NMR (D₂O) *δ*168,0; 147,6; 140,3; 130,7; 127,9; 125,0; 122,6; 56,8; 32,6; 30,1; 29,9; 28,2; 24,8; 19,9. Anal. (C₁₄H₁₈N₂,HCl) C, H, N.

### Chlorhydrate de (2,3-Dimethyl-phenyl)-(5-methyl-4,5-dihydro-3H-pyrrol-2-yl)-amine (6).

¹H NMR (D₂O) *δ* 7.24 (d, 1H, Har, J= 7.6); 7.17 (t, 1H, Har, J= 7.7); 7.06 (d, 1H, Har, J= 7.6); 4.02 (h, 1H, CH, J= 6.4); 3.09-3.01 (m, 2H, CH₂); 2.39-2.29 (m, 1H, 1/2 CH₂); 2.24 (s, 3H, CH₃); 2.08 (s, 3H, CH₃); 1.85-1.78 (m, 1H, 1/2 CH₂); 1.13 (d, 3H, CH₃, J= 6.4). ¹³C NMR (D₂O) *δ* 168.2; 139.2; 133.5; 133.1; 131.2; 126.6; 124.0; 56.2; 43.8; 37.0; 28.6; 24.9; 17.2. Anal. (C₁₃H₁₈N₂.HCl) C, H, N.

### Chlorhydrate de Indan-4-yl-(4-méthyl-4,5-dihydro-3H-pyrrol-2-yl)-amine (7).

¹H NMR (D₂O) *δ* 7,28 (d, 1H, Har, J= 7,5); 7,21 (t, 1H, Har, J= 7,6); 7,03 (d, 1H, Har, J= 7,5); 3,70 (dd, 1H, 1/2 CH₂, J= 10,8, J= 7,8); 3,19 (dd, 1H, 1/2 CH₂, J= 11, J= 6); 3,17-3,11 (m, 1H, 1/2 CH₂); 2,89 (t, 2H, CH₂, J= 7,4); 2,74 (t, 2H, CH₂, J= 7,4); 2,68-2,61 (m, 2H, CH + 1/2 CH₂); 2,01 (qn, 2H, CH₂, J= 7,5); 1,10 (d, 3H, CH₃, J= 7). ¹³C NMR (D₂O) *δ* 168,3; 147,6; 140,2; 130,5; 127,9; 124,9; 122,5; 54,1; 37,8; 32,6; 29,9; 29,5; 24,7; 17,6. Anal. (C₁₄H₁₈N₂,HCl) C, H, N

### Chlorhydrate de (7-Chloro-indan-4-yl)-(4-méthyl-4,5-dihydro-3H-pyrrol-2-yl)-amine (8).

¹H NMR (D₂O) *δ* 7,22 (d, 1H, Har, J= 8,3); 7,01 (d, 1H, Har, J= 8,4); 3,70 (dd, 1H, 1/2 CH₂, J= 10,9, J= 7,7); 3,20 (dd, 1H, 1/2 CH₂, J= 10,9, J= 7,2); 3,15-3,09 (m, 1H, 1/2 CH₂); 2,93 (t, 2H, CH₂, J= 7,6); 2,82 (t, 2H, CH₂, J= 7,5); 2,69-2,61 (m, 2H, CH + 1/2 CH₂); 2,04 (qn, 2H, CH₂, J= 7,6); 1,08 (d, 3H, CH₃, J= 6,8). ¹³C NMR (D₂O) *δ*168,5; 145,2; 142,4; 130,4; 129,1; 127,8; 124,5; 54,2; 37,9; 32,2; 30,9; 29,5; 23,8; 17,7. Anal. (C₁₄H₁₇ClN₂,HCl) C, H, N.

### Chlorhydrate de (7-Méthyl-indan-4-yl)-(4-méthyl-4,5-dihydro-3H-pyrrol-2-yl)-amine (9).

¹H NMR (D₂O) *δ* 7,06 (d, 1H, Har, J= 8); 6,95 (d,1H, Har, J= 8); 3,7 (dd, 1H, 1/2 CH₂, J= 10,8, J= 7,7); 3,21-3,09 (m, 2H, 1/2 CH₂ + CH); 2,82 (t, 2H, CH2, J= 7,5); 2,75 (t, 2H, CH₂, J= 7,5); 2,70-2,64 (m, 2H, CH₂); 2,19 (s, 3H, CH₃); 2,01 (qn, 2H, CH₂, J= 7,5); 1,09 (d, 3H, CH₃, J= 6,7). ¹³C NMR (D₂O) *δ*168,4; 145,9; 139,8; 135,2; 132,4; 128,5; 128,1; 122,8; 54,0; 37,8; 31,3; 30,1; 29,6; 24,2; 18,1; 17,7. Anal. (C₁₅H₂₀N₂,HCl) C, H, N.

### Chlrohydrate de 1-(3,4-dihydro-2H-pyrrol-5-yl)-2-méthylindoline (10).

¹H-NMR (400 MHz, MeOH-d4) *δ* 7,44-7,29 (m, 4H); 4,74 (m, 1 H); 3,86 (m, 2 H); 6,62-3,56 (m, 2 H); 3,29-3,23 (1 H); 2,88-2,84 (m, 1 H); 2,41-2,32 (m, 2 H); 1,35 (d, 3 H, J = 5 Hz). C-NMR δ (100 MHz, MeOH-d4 δ 116,7; 140,5; 134,5; 129,3; 128,0; 127,9; 117,0; 61,8;
37,0; 33,1; 22,1; 19,3.
MS-ESI (m/z): [M+H]⁺ calc. pour C₁₃H₁₇N₂: 201. Trouvé: 201

### Chlorhydrate de 7-méthyl-1-(3-méthyl-3,4-dihydro-2H-pyrrol-5-yl)indoline (11).

¹H-NMR (400 MHz, MeOH-d4) *δ* 7,28-7,20 (m, 3 H); 4,282-4,24 (m, 2 H); 3,98-3,93 (m, 1 H); 3,46-3,39 (m, 2 H); 3,23-3,20 (m, 2 H); 2,95-84 (m, 2 H); 2,29 (s, 3 H); 1,26 (d, 3 H, J = 8 Hz). ¹³C-NMR δ (100 MHz, MeOH-d4) δ 168,4; 140,1; 137,8; 131,9; 129,5; 128,8; 124,3; 57,0, 56,2; 54,9; 41,2; 31,6; 31,0; 19,4; 18,7.
MS-ESI (m/z): [M+H]⁺ calc. pour C₁₃H₁₉N₂: 215. Trouvé: 215.

### Chlorhydrate de 2-méthyl-1-(3-méthyl-3,4-dihydro-2H-pyrrol-5-yl)indoline (mélange de diastéréoisomères) (12).

Huile, ¹H-NMR (400 MHz, acétone-d6) δ 7,43-7,17 (m, 4 H); 5,30-5,28 (m, 1 H); 3,96-3,85 (m, 2 H); 3,53-327 (m, 3 H); 2,91-2,74 (m, 3 H); 1,32-1,04 (m, 6 H).

¹³C-NMR δ (100 MHz, acétone-d6) δ 164,9 (164,4); 140,7; 134,2; 128,7; 127,4; 116,7 (116,6); 61,2 (61,1); 40,6 (40,4); 31,7; 30,4); 25,7; 19,9 (19,8); 18,0
MS-ESI (m/z): [M+H]⁺ calc for C₁₃H₁₉N₂: 215. Trouvé: 215.

### Chlorhydrate de (2-Chlorophényl)-(5-méthyl-4,5-dihydro-3H-pyrrol-2-yl)-amine (13). (non compris dans l'invention)

mp 173-4 °C. ¹H NMR (D₂O) *δ*7.67 (m, 1H, H ar); 7,50-7,53 (m, 3H, Har); 4,21 (m, 1H, CH); 3,18 (m, 2H, CH₂); 2,51 (m, 1H, 1/2 CH₂); 1,94 (m, 1H, 1/2 CH₂); 1,30 (d, 3H, CH₃). ¹³C NMR (D₂O) *δ* 168,1; 132,5; 131,4; 131,3; 131,0; 129,3; 128,6; 57,9; 30,9; 28,8; 20,5. Anal. (C₁₁H₁₃ClN₂,HCl) C, H, N.

### Chlorhydrate de (2-Fluoro-5-méthyl-phényl)-(5-méthyl-4,5-dihydro-3H-pyrrol-2-yl)-amine (14). (non compris dans l'invention

¹H NMR (D₂O) *δ* 7,20-7,09 (m, 3H, Har); 4,11 (hex, 1H, CH, J= 6,8); 3,09-2,98 (m, 2H, CH₂); 2,42-2-37 (m, 1H, 1/2 CH₂); 2,25 (s, 3H, CH₃); 1,85-1,73 (m, 1H, 1/2 CH₂); 1,08 (d, 3H, CH₃, J= 6,5). ¹³C NMR (D₂O) *δ*168,4; 154,3 (d, J¹_{C-F}= 244,5); 135,7; 130,9; 126,9; 121,6 (d, J²_{C-F}= 13,2); 116,4 (d, J²_{C-F}= 19,5); 57,2; 30,5; 28,1; 21,3; 19,7. ¹⁹F NMR (D₂O) *δ* -128,3. Anal. (C₁₂H₁₅FN₂,HCl) C, H, N.

### Chlorhydrate de (2-Chloro-5-méthyl-phényl)-(5-méthyl-4,5-dihydro-3H-pyrrol-2-yl)-amine (15). (non compris dans l'invention

¹H NMR (D₂O) *δ*7,38 (dd, 1H, Har, J= 3,1, J= 7,8); 7,18 (d, 1H, Har, J= 2,9); 7,16 (dd, 1H, Har, J= 3,0, J= 7,7); 4,09-3,96 (m, 1H, CH); 3,07-2,94 (m, 2H, CH₂); 2,41-2,33 (m, 1H, 1/2 CH₂); 2,24 (s, 3H, CH₃); 1,82-1,72 (m, 1H, 1/2 CH₂); 1,16 (d, 3H, CH₃, J= 6,3). ¹³C NMR (D₂O) *δ* 168,5; 139,4; 131,2, 130,2; 128,0; 126,8; 124,4; 57,1; 30,2; 28,1; 19,8; 19,7. Anal. (C₁₂H₁₅ClN₂,HCl) C, H, N

### Chlorhydrate de (4-Fluoro-2-méthyl-phényl)-(5-méthyl-4,5-dihydro-3H-pyrrol-2-yl)-amine (16). (non compris dans l'invention

¹H NMR (D₂O) *δ* 7,22 (dd, 1H, Har, J= 5,4, J= 8,7); 7,09 (dd, 1H, Har, J= 2,9, J= 8,7); 7,0 (dt, 1H, Har, J= 2,9, J= 8,8); 4,14 (hex, 1H, CH, J= 6,7); 3,11-3,03 (m, 2H, CH₂); 2,45-2,97 (m, 1H, 1/2 CH₂); 2,17 (s, 3H, CH₃); 1,81-1,74 (m, 1H, 1/2 CH₂); 1,11 (d, 3H, CH₃, J= 6,4). ¹³C NMR (D₂O) *δ*169,8; 161,9 (d, J¹_{C-F}= 248,8); 137,8 (d, J³_{C-F}= 9,2); 129,3 (d, J⁴_{C-F}= 2,8); 128,3 (d, J³_{C-F}= 9,6); 117,5 (d, J²_{C-F}= 22,7); 114,2 (d, J²_{C-F}= 23,0); 55,6; 31,5; 28,8; 19,7; 19,5. ¹⁹F NMR (D₂O) *δ* -113,4. Anal. (C₁₂H₁₅FN₂,HCl) C, H, N.

### Chlorhydrate de (5,6,7,8-tetrahydro-naphthalen-1-yl)-(5-Methyl-4,5-dihydro-3H-pyrrol-2-yl)-amine (17). (non compris dans l'invention

¹H NMR (D₂O) *δ* 7.17 (d, 2H, Har, J= 7); 7.03 (t, 1H, Har, J= 7.1); 4.06-4.00 (m, 1H, CH); 3.04-2.97 (m, 2H, CH₂); 2.72 (t, 2H, CH₂, J= 5.7); 2.51 (t, 2H, CH₂, J= 5.9); 2.40-2.32 (m, 1H, 1/2 CH₂); 1.81-1.61 (m, 5H, 1/2 CH₂ + 2xCH₂); 1.15 (d, 3H, CH₃, J= 6.4). ¹³C NMR (D₂O) *δ*168.6; 140.1; 133.9; 133.0; 130.2; 126.6; 123.6; 56.6; 29.9; 28.9; 28.2; 24.1; 22.1; 22.0; 19.8. Anal. (C₁₅H₂₀N₂.HCl) C, H, N.

### Chlorhydrate de Indan-5-yl-(5-méthyl-4,5-dihydro-3H-pyrrol-2-yl)-amine (18). (non compris dans l'invention

¹H NMR (D₂O) *δ* 7,26 (d, 1H, Har, J= 8,0); 7,08 (d, 1H, Har, J= 1,5); 6,96 (dd, 1H, Har, J= 1,9, J= 7,9); 4,03 (h, 1H, CH, J= 6,6); 2,99-2,92 (m, 2H, CH₂); 2,80 (t, 4H, 2xCH₂, J= 7,5); 2,35-2,29 (m, 1H, 1/2 CH₂); 1,97 (qn, 2H, CH₂, J= 7,5); 1,79-1,71 (m, 1H, 1/2 CH₂); 1,16 (d, 3H, CH₃, J= 6,4). ¹³C NMR (D₂O) *δ* 167,7; 145,7; 145,1; 132,7; 125,5; 121,9; 120,1; 56,9; 32,3; 32,0; 30,4; 28,0; 25,3; 19,8. Anal. (C₁₄H₁₈N₂,HCl) C, H, N.

### Chlorhydrate de (6-Chloro-indan-5-yl)-(5-méthyl-4,5-dihydro-3H-pyrrol-2-yl)-amine (19). (non compris dans l'invention

¹H NMR (D₂O) *δ* 7,18 (s, 1H, Har); 6,91 (s, 1H, Har); 4,07 (h, 1H, CH, J= 6,6); 2,97-2,90 (m, 2H, CH₂); 2,81 (t, 4H, 2xCH₂, J= 7,5); 2,36-2,28 (m, 1H, 1/2 CH₂); 1,97 (qn, 2H, CH₂, J= 7,5); 1,79-1,71 (m, 1H, 1/2 CH₂); 1,63 (d, 3H, CH₃, J= 6,4). ¹³C NMR (D₂O) *δ* 168,5; 145,3; 145,9; 133,4; 127,5; 122,7; 121,6; 57,2; 32,2; 31,8; 30,6; 27,9; 25,5; 21,1. Anal. (C₁₄H₁₇ClN₂,HCl) C, H, N.

### Chlorhydrate de (7-Chloro-indan-4-yl)-(5-méthyl-4,5-dihydro-3H-pyrrol-2-yl)-amine (20).

¹H NMR (D₂O) *δ* 7,22 (d, 1H, Har, J= 8,3); 7,01 (d, 1H, Har, J= 8,4); 4,05 (h, 1H, CH, J= 6,4); 3,05-2,99 (m, 2H, CH₂); 2,93 (t, 2H, CH₂, J= 7,6); 2,81 (t, 2H, CH₂, J= 7,5); 2,40-2,32 (m, 1H, 1/2 CH₂); 2,03 (qn, 2H, CH₂, J= 7,6); 1,82-1,77 (m, 1H, 1/2 CH₂); 1,17 (d, 3H, CH₃, J= 6,4). ¹³C NMR (D₂O) *δ*168,4; 145,1; 142,5; 130,5; 129,2; 127,8; 124,4; 56,8; 32,6; 30,1; 29,9; 28,2; 24,8; 19,9. Anal. (C₁₄H₁₈ClN₂,HCl) C, H, N.

### Chlorhydrate de (2,3-Diméthyl-phényl)-(4-méthyl-4,5-dihydro-3H-pyrrol-2-yl)-amine (21).

¹H NMR (D₂O) *δ* 7,21 (d, 1H, Har, J= 7,7); 7,16 (t, 1H, Har, J= 7,7); 7,03 (d, 1H, Har, J= 7,6); 3,71-3,65 (m, 1H, 1/2 CH₂); 3,23-3,17 (m, 2H, 1/2 CH₂ + CH); 2,74-2,70 (m, 2H, CH₂); 2,30 (s, 3H, CH₃); 2,11 (s, 3H, CH₃); 1,12 (s, 6H, 2xCH₃). ¹³C NMR (D₂O) *δ*168,7; 139,5; 133,4; 132,8; 130,5; 126,8; 123,7; 54,5; 43,7; 37,0; 25,6; 19,4; 12,3. Anal. (C₁₃H₁₈N₂,HCl) C, H, N.

### Chlorhydrate de Indan-5-yl-(4-méthyl-4,5-dihydro-3H-pyrrol-2-yl)-amine (22). (non compris dans l'invention

¹H NMR (D₂O) *δ* 7,26 (d, 1H, Har, J= 8,0); 7,08 (d, 1H, Har, J= 1,5); 6,96 (dd, 1H, Har, J= 1,9, J= 7,9); 4,03 (h, 1H, CH, J= 6,6); 2,97-2,93 (m, 2H, CH₂); 2,80 (t, 4H, 2xCH₂, J= 7,5); 2,35-2,29 (m, 1H, 1/2 CH₂); 1,97 (qn, 2H, CH₂, J= 7,5); 1,79-1,71 (m, 1H, 1/2 CH₂); 1,16 (d, 3H, CH₃, J= 6,4). ¹³C NMR (D₂O) *δ* 167,7; 145,7; 145,1; 132,7; 125,5; 121,9; 120,1; 56,9; 32,3; 32,0; 30,4; 28,0; 25,3; 19,8. Anal. (C₁₄H₁₈N₂,HCl) C, H, N.

### Chlorhydrate de (5,6,7,8-tétrahydro-naphthalen-1-yl)-(4-Méthyl-4,5-dihydro-3H-pyrrol-2-yl)-amine (23). (non compris dans l'invention

¹H NMR (D₂O) *δ*7,17 (d, 2H, Har, J= 7); 7,03 (t, 1H, Har, J= 7,1); 4,07-3,99 (m, 1H, CH); 3,04-2,96 (m, 2H, CH₂); 2,72 (t, 2H, CH₂, J= 5,7); 2,51 (t, 2H, CH₂, J= 5,9); 2,39-2,33 (m, 1H, 1/2 CH₂); 1,81-1,61 (m, 5H, 1/2 CH₂ + 2xCH₂); 1,15 (d, 3H, CH₃, J= 6,4). ¹³C NMR (D₂O) *δ* 168,6; 140,1; 133,9; 133,0; 130,2; 126,6; 123,6; 56,6; 29,9; 28,9; 28,2; 24,1; 22,1; 22,0; 19,8. Anal. (C₁₅H₂₀N₂,HCl) C, H, N.

### Chlorhydrate de Indan-4-yl-(5,5-diméthyl-4,5-dihydro-3H-pyrrol-2-yl)-amine (24). (non

**compris dans l'invention** ¹H NMR (D₂O) *δ* 7,27 (t, 1H, Har, J= 8); 7,19 (d, 1H, Har, J= 7,5); 7,01 (d, 1H, Har, J= 7,6); 3,08 (t, 2H, CH₂, J= 7,9); 2,88 (t, 2H, CH₂, J= 7,5); 2,76 (t, 2H, CH₂, J= 7,6); 2,13-1,95 (m, 4H, 2xCH₂); 1,27 (s, 6H, 2xCH₃). ¹³C NMR (D₂O) *δ* 166,7; 147,5; 140,3; 130,6; 127,9; 125,0; 122,7; 64,7; 34,3; 32,6; 30,0; 29,7; 26,8; 24,8. Anal. (C₁₅H₂₁ClN₂,HCl) C, H, N.

### Chlorhydrate de (3-Chloro-2-méthyl-phényl)-(5,5-diméthyl-4,5-dihydro-3H-pyrrol-2-yl)-amine (25). (non compris dans l'invention

¹H NMR (D₂O) *δ* 7,40 (d, 1H, Har, J= 7,9); 7,21 (d, 1H, Har, J= 7,9); 7,18 (t, 1H, Har, J= 7,8); 3,08 (t, 2H, CH₂, J= 7,6); 2,19 (s, 3H, CH₃); 1,95 (t, 2H, CH₂, J= 7,8); 1,26 (s, 6H, 2xCH₃). ¹³C NMR (D₂O) *δ* 166,7; 135,5; 133,4; 130,1; 128,1; 125,3; 65,0; 37,4; 29,6; 26,8; 24,9. Anal. (C₁₃H₁₇ClN₂,HCl) C, H, N.

### Chlorhydrate de (2,3-Diméthyl-phényl)-(4,4-diméthyl-4,5-dihydro-3H-pyrrol-2-yl)-amine (26). (non compris dans l'invention

¹H NMR (D₂O) *δ* 7,24 (d, 1H, Har, J= 7,6); 7,17 (t, 1H, Har, J= 7,7); 7,06 (d, 1H, Har, J= 7,6); 3,32 (s, 2H, CH₂); 2,87 (s, 2H, CH₂); 2,25 (s, 3H, CH₃); 2,09 (s, 3H, CH₃); 1,16 (s, 6H, 2xCH₃). ¹³C NMR (D₂O) *δ* 168,7; 139,5; 133,4; 132,8; 130,5; 126,8; 123,7; 59,2; 43,7; 37,0; 25,6; 19,4; 12,3. Anal. (C₁₄H₂₀N₂,HCl) C, H, N.

### Chlorhydrate de (3-Chloro-2-méthyl-phényl)-(4,4-diméthyl-4,5-dihydro-3H-pyrrol-2-yl)-amine (27). (non compris dans l'invention

¹H NMR (D₂O *δ* 7,45 (d, 1H, Har, J= 7,9); 7,25 (d, 1H, Har, J= 7,9); 7,20 (t, 1H, Har, J= 7,8); 3,70 (m, 1H, 1/2 CH₂); 3,19 (m, 2H, 1/2 CH₂ + CH); 2,74 (m, 2H, CH₂); 2,22 (s, 3H, CH₃); 1,10 (d, 3H, CH₃, J= 6,5). ¹³C NMR (D₂O) *δ* 169,1; 135,5; 133,4; 130,1; 128,1; 125,3; 54,2; 37,9; 29,5; 18,1; 14,2. Anal. (C₁₃H₁₇ClN₂,HCl) C, H, N.

### DESCRIPTION DES FIGURES

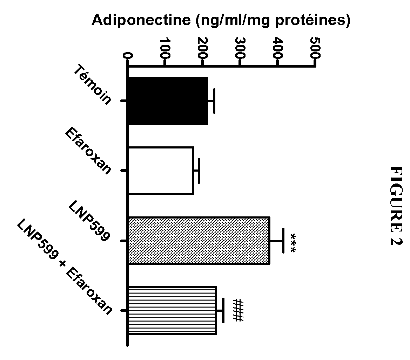

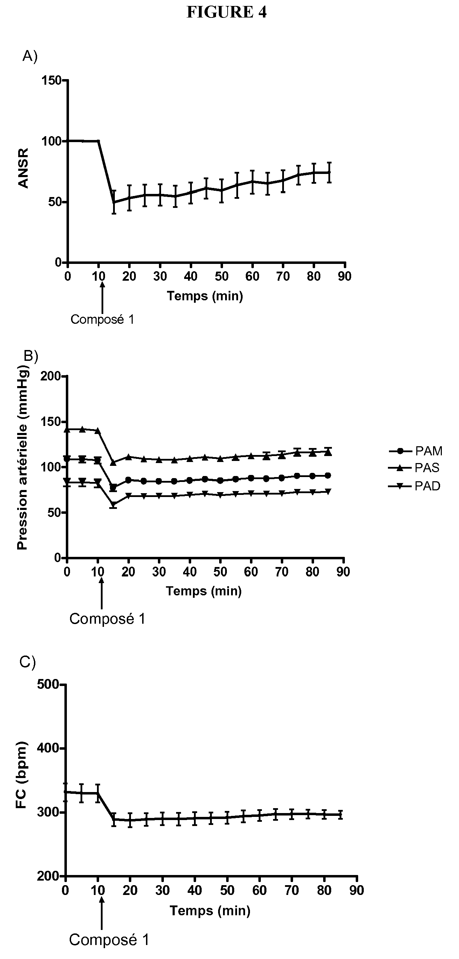

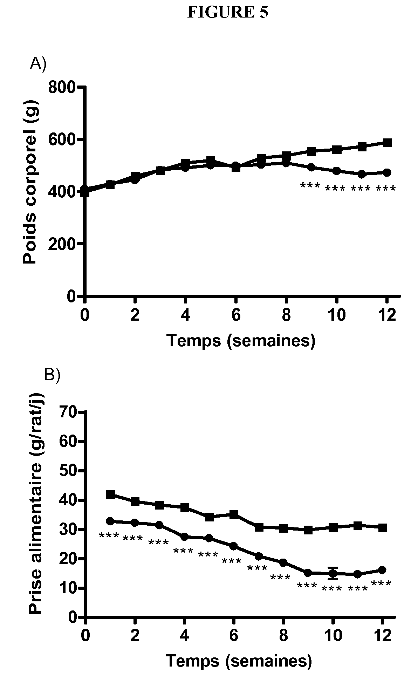

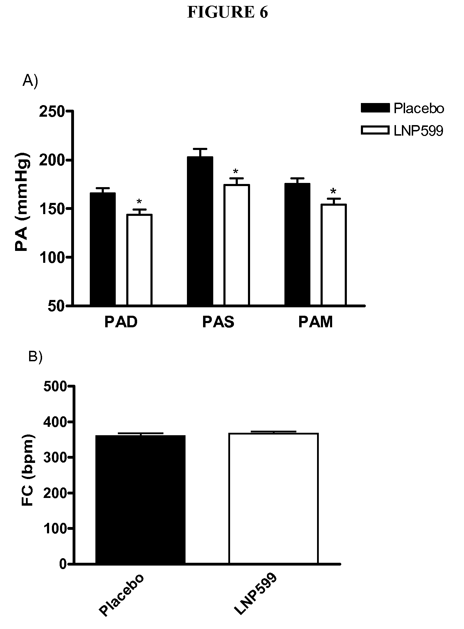

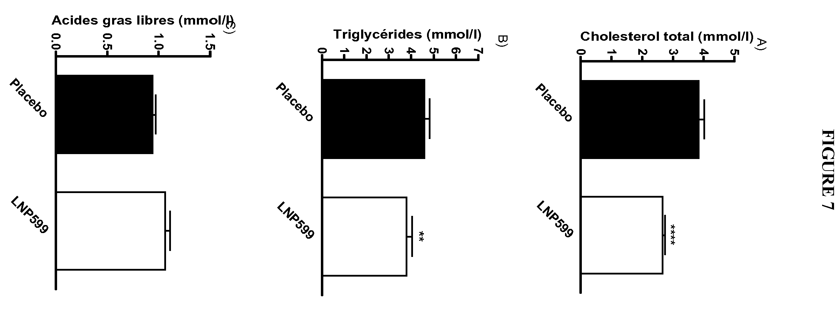

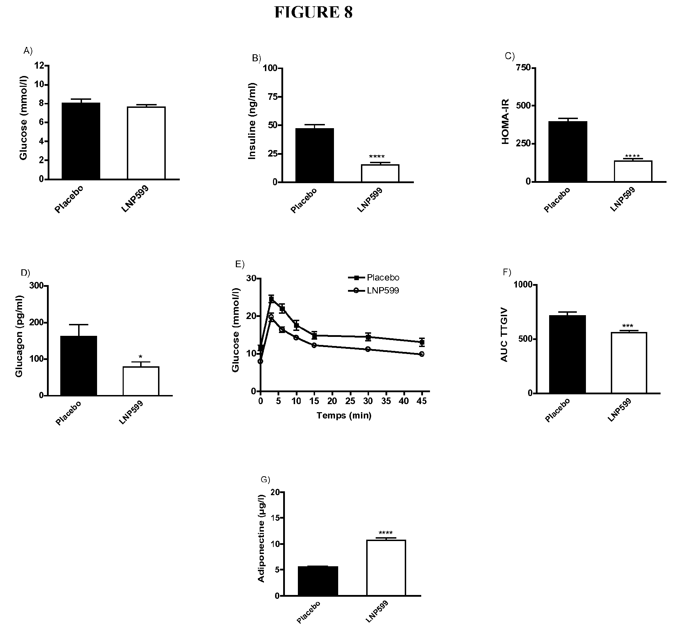

**La** **figure 1A** présente les études de compétition de la liaison à [¹²⁵I]-PIC dans des membranes de cellules 3T3 et **1B** présente les études de compétition de la liaison à [¹²⁵I]-PIC dans des membranes de cellules PC12 réalisées conformément à l'exemple 1.3. Chaque point est la moyenne de 2 à 4 expériences réalisées en triple.
**Figure 1A** : l'axe des ordonnées représente la liaison spécifique à [¹²⁵I] PIC et l'axe des abscisses représente le log [M] en clonidine (carré) et composé 1 selon l'invention (triangles).
**Figure 1B** **:** l'axe des ordonnées représente la liaison spécifique à [¹²⁵I] PIC et l'axe des abscisses représente le log [M] du composé 1.
**La** **figure 2** présente l'effet du composé 1 selon l'invention à la dose de 3 µmoles/l sur la sécrétion d'adiponectine mesuré en l'absence ou en présence d'efaroxan à la dose de 100 µmoles/l conformément à l'exemple 1.4.
   *** p<0, 001 par rapport au témoin ; ### p<0, 001 par rapport au composé 1.
   L'axe des ordonnées représente la sécrétion en adiponectine exprimée en ng/ml/mg protéines.
   L'axe des abscisses représente de gauche à droite : histogramme noir : groupe témoin, histogramme blanc : groupe traité par Efaroxan (100 µmol/ml), histogramme gris : groupe traité par le composé 1 (3 µmol/ml), et histogramme hachuré : groupe traité par le composé 1 (3 µmol/ml) + Efaroxan (100 µmol/ml).
**La** **figure 3** **représente la liste des systèmes sur lesquels le déplacement de la liaison spécifique** par le composé 1 a été mesuré selon l'exemple 3.
**Les** **figures 4A à 4C** présentent les effets d'un traitement aigu par le composé 1 à une dose de 10 mg/kg (i.v.) sur les paramètres hémodynamiques et l'activité sympathique de rats Sprague-Dawley anesthésiés à l'uréthane (1,5 g/kg, i. p.) conformément à l'exemple 4.
**La** **figure 4A** présente la variation de l'activité rénale sympathique en fonction du temps.
   Axe des ordonnées: mesure de l'activité sympathique rénale.
   Axe des abscisses: temps en minutes (la flèche montre le temps d'administration du composé 1).
**La** **figure 4B** présente la variation de pression artérielle en mm Hg en fonction du temps. Les cercles noirs représentent la pression artérielle moyenne ; les losanges noirs pointes en haut représentent la pression artérielle systolique et les losanges noirs pointes en bas représentent la pression artérielle diastolique.
   Axe des ordonnées: mesure de la pression artérielle moyenne (PAM) systolique (PAS) et diastolique (PAD).
   Axe des abscisses: temps en minutes (la flèche montre le temps d'administration du composé 1).
**La** **figure 4C** présente la variation de fréquence cardiaque (FC) en bpm en fonction du temps.
   Axe des ordonnées : fréquence cardiaque (HR) exprimé en battements par minute (bpm)
   Axe des abscisses: temps en minutes (la flèche montre le temps d'administration du composé 1).
**Les** **figures 5A** et **5B** présentent les effets d'un traitement chronique par le composé 1 (20 mg/kg/j dans l'eau de boisson) sur le poids corporel **(****figure 5A****)** et la consommation de nourriture **(****figure 5B****),** chez des rats SHHF (*spontaneously hypertensive*, *heart failure; spontanément hypertendu*, *insuffisante cardiaque*), durant les 12 semaines de traitement mesurés conformément à l'exemple 5.
**Figure 5A** **:** ordonnées : poids corporel en grammes ; abscisses : temps en semaines.
   Les carrés noirs représentent les témoins et les cercles noirs représentent le groupe traité avec le composé 1. ***: p<0,001 (ANOVA 2 facteurs).
**Figure 5B**: ordonnées : consommation de nourriture en grammes/rat/jour; abscisses: temps en semaines.
   Les carrés noirs représentent les témoins et les cercles noirs représentent le groupe traité avec le composé 1. ***: p<0,001 (ANOVA 2 facteurs).
**Les** **figures 6A** et **6B** présentent les effets de 12 semaines de traitement par le composé 1 (20 mg/kg/j dans l'eau de boisson) sur la pression artérielle (figure 6A) et la fréquence cardiaque (figure 6B) chez des rats SHHF mesurés conformément à l'exemple 5.
   A la fin du traitement, les rats SHHF ont été anesthésiés avec du pentobarbital sodique (50 mg/kg/i,p,) trachéotomisés et ventilés avec l'air ambiant.
   La pression artérielle et la fréquence cardiaque ont été enregistrées grâce à un cathéter inséré dans l'artère fémorale gauche.
**Figure 6A****:** ordonnées : Pression artérielle en mm Hg ; de gauche à droite : pression artérielle diastolique (PAD), pression artérielle systolique (PAS) et pression artérielle moyenne (PAM). Les histogrammes en noir représentent le groupe témoin, les histogrammes en blanc représentent le groupe traité avec le composé 1. *: p<0,05 (test t de Student).
**Figure 6B****:** ordonnées : fréquence cardiaque (FC) exprimée en battements par minute (bpm) ; l'histogramme en noir représente le groupe témoin, l'histogramme en blanc représente le groupe traité avec le composé 1.
**Les** **figures 7A** à **7C** représentent les effets d'un traitement de 12 semaines avec le composé 1 (20 mg/kg/j dans l'eau de boisson) sur le cholestérol total plasmatique (figure 7A), les triglycérides (figure 7B) et les acides gras libres (figure 7C) dans le plasma_des rats SHHF mesurés conformément à l'exemple 5.
   A la fin du traitement, les rats SHHF ont été anesthésiés avec de l'isoflurane (2,5%) après un jeûne de 18h et des échantillons sanguins ont été obtenus à partir de la veine caudale.
**Figure 7A** **:** ordonnées : cholestérol total (mmol/l); histogramme noir : groupe témoin et histogramme blanc: groupe traité avec le composé 1. ****: p<0,0001 (test t de Student).
**Figure 7B****:** ordonnées : triglycérides (mmol/l); histogramme noir : groupe témoin et histogramme blanc: groupe traité avec le composé 1. **: p<0,01;
**Figure 7C** **:** ordonnées : acides gras libres (mmol/l); histogramme noir : groupe témoin et histogramme blanc : groupe traité avec le composé 1.
**Les** **figures 8A à 8G** représentent les effets du traitement de 12 semaines avec le composé 1 (20 mg/kg/j dans l'eau de boisson) sur le métabolisme du glucose chez des rats SHHF mesurés conformément à l'exemple 5.
   A la fin du traitement, les rats SHHF ont été anesthésiés avec du pentobarbital sodique (50 mg/kg, i.p.) après 18 h de jeûne. La veine fémorale gauche a été cathétérisée pour prélever les échantillons sanguins pour les différents dosages. Pour effectuer le test de tolérance au glucose, une solution de glucose (0,5g/kg, iv) a été administrée et les concentrations plasmatiques de glucose ont été déterminées après 3, 6, 10, 15, 30 et 45 minutes.
**Figure 8A****:** ordonnées : glucose (mmol/l); histogramme noir : groupe témoin, histogramme blanc: groupe traité avec composé 1.
**Figure 8B****:** ordonnées : insuline (ng/ml) ; histogramme noir : groupe témoin, histogramme blanc: groupe traité avec composé 1. ****: p<0,0001 (test t de Student).
**Figure 8C**: ordonnées : (indice HOMA d'insulino-résistance) ; histogramme noir : groupe témoin, histogramme blanc : groupe traité avec composé 1. ****: p<0,0001 (test t de Student).
**Figure 8D****:** ordonnées : glucagon ; histogramme noir : groupe témoin, histogramme blanc : groupe traité avec composé 1. *: p<0,05 (test t de Student).
**Figure8E****:** ordonnées : adiponectine (µg/ml); histogramme noir : groupe témoin, histogramme blanc : groupe traité avec le composé 1. ****: p<0,0001 (test t de Student).
**Figure 8F** **:** ordonnées : temps en minutes (carrés noirs : groupe témoin, cercles blancs: groupe traité avec le composé 1.
**Figure 8G****:** ordonnées : Aire sous la courbe (AUC) après le test de tolérance au glucose; histogramme noir : groupe témoin, histogramme blanc : groupe traité avec le composé 1.
   ***: p<0,001 (test t de Student).
**Figure 9** donne les effets pharmacologiques des composés 1-27 de l'invention sur la pression artérielle moyenne (PAM) et sur la fréquence cardiaque (FC) chez le rat normotendu anesthésié mesurés conformément à l'exemple 6.

### PARTIE PHARMACOLOGIQUE

### EXEMPLE 1: Expériences in vitro

### 1.1 Culture des cellules

Les préadipocytes murins 3T3-L1 ont été cultivés à confluence à 37°C dans du milieu DMEM contenant 4,5 g/litre de D-glucose, 10 % FCS, et des antibiotiques. A confluence, la différenciation des adipocytes 3T3-L1 a été initiée par addition pendant 48 h d'un mélange contenant 100 µM méthyl-isobutylxanthine, 100 nM dexamethasone, et 175 nM insuline. Les cellules ont été ensuite repiquées à nouveau tous les 2-3 jours avec du DMEM, 10 % de FCS et 175 nM d'insuline. Plus de 95 % des cellules ont le phénotype d'adipocytes matures, 10 jours après que la confluence ait été atteinte.

Les cellules PC-12 sont cultivées dans des boites 75-cm² dans un milieu DMEM (1000 mg/l glucose) complémenté avec 10% de FBS inactivé par traitement à 56°C, 100 U/ml pénicilline et 100 µg/ml streptomycine. Quand les cellules ont atteint la confluence (3 à 4 jours après le début de la culture), elles sont récoltées et repiquées par action de 0,25% de trypsine durant 2 minutes à 37°C. Pour les études de liaison spécifique, le milieu est retiré et les cellules sont conservées par congélation à - 20°C jusqu'à utilisation pour la préparation membranaire.

### 1.2 Préparations membranaires et extraits cellulaires

Les adipocytes ont été lavés deux fois avec du PBS glacé, récoltés et homogénéisés dans du tampon Tris-HCl 25 mM, pH 7,5, EDTA 1 mM. Les homogénats ont été centrifugés à 20,000 *x g* for 15 min à 4°C, et le surnageant a été conservé à -80°C jusqu'à utilisation. Les culots ont été resuspendus dans du tampon Tris-HCl 25 mM, pH 7,5, EDTA 1 mM, et stockés à -80°C. Des aliquotes des homogénats et surnageants ont été utilisées pour déterminer le contenu protéique (BC Assay Uptima kit, Interchim, Montluçon, France), en utilisant la protéine BSA comme standard.

Les cellules de PC12, congelées, sont récupérées par grattage dans un tampon Tris-HEPES glacé (5 mM Tris-HEPES, pH 7.7, 0.5 mM EDTA, 0.5 mM EGTA, and 0.5 mM MgCl₂) et homogénéisé avec un Potter. Après centrifugation à 75,000g pendant 20 min, le culot est lavé avec un tampon Tris-HEPES glacé puis centrifugé à nouveau. Cette dernière opération est réalisée deux fois. Les culots sont resuspendus dans le même tampon à une concentration de 1 à 2 mg de protéines / ml. Les préparations membranaires sont conservées à -80°C jusqu'à utilisation.

### 1.3 Test de liaison avec le composé 1 sur des préparations membranaires d'adipocytes et les cellules de PC12

### 1.3.1. Mode opératoire

Les tests de liaison spécifique en compétition ont été effectués en utilisant 0,5 nM [¹²⁵I]-PIC en présence de 10µM rauwolscine pour les membranes de cellules 3T3 ou en absence de rauwolscine pour les cellules PC12 et 6 concentrations différentes du ligand à étudier, de 10⁻⁹ à 10⁻⁴M.

L'incubation a été initiée par addition de membranes de cellules 3T3 (10-26 µg de protéines) ou de cellules PC12 (10-25 µg of protéines) dans un volume final de 250 µl de tampon Tris-HEPES (50mM Tris-HEPES, pH 7,7, 0,5mM EDTA, 0,5mM EGTA et 0,5mM MgCl₂) et a été effectuée à 25°C durant 45 min.

La réaction a été arrêtée par filtration rapide sous vide à travers des filtres en fibre de verre GF/B traités par PEI 0,3% avec un appareil de filtration de type Brandel® suivi par trois lavages rapides des filtres avec 3 ml de tampon Tris-HCl 50mM glacé, pH 7,4. La radioactivité retenue sur les filtres séchés a été déterminée par un compteur gamma Minaxi (Packard, Meriden, CT, U,S,A,). La liaison non spécifique a été déterminée par la liaison de [¹²⁵I]PIC en présence de 10 µM PIC, et représente environ 43 % du total de la radioactivité. Le choix de 10 µM PIC vient d'expérience pilotes montrant qu'à cette concentration, la liaison résiduelle obtenue avec le PIC était similaire à celle obtenue avec la clonidine.

### 1.3.2. Résultats

Les résultats sont présentés sur la figure 1.

La liaison spécifique de 0,5 nM [¹²⁵I]-PIC a été mesurée. Elle est comprise de 2633 à 6652 cpm dans la préparation membranaire d'adipocytes 3T3 et de 2100 à 3400 cpm dans la préparation membranaire de cellules de PC12.

Dans les préparations membranaires d'adipocytes (3T3), la clonidine, molécule de référence pour le RI₁, est capable de déplacer la liaison spécifique de [¹²⁵I]-PIC dans la préparation de membranes cellulaires 3T3 avec deux affinités (CI₅₀ = 54,9 ± 5,4 nM (57 % des sites totaux) et 8144 ± 426 nM, n=2) démontrant la présence du récepteur I₁ dans la préparation de membranes 3T3-L1. De plus, le composé 1 sélectif déplace la liaison spécifique de [¹²⁵I]-PIC sur le récepteur I₁ sur un site de haute affinité (CI₅₀ = 104 ± 7 nM (n=4) (figure 1A)).

Dans les préparations membranaires de cellules de PC12, le composé 1 sélectif du récepteur I₁ déplace la liaison spécifique de [¹²⁵I]-PIC avec deux affinités (haute affinité CI₅₀ = 3,2 + 0,7 nM (55%) et faible affinité 30698 + 5433 nM n=2) comme montré dans la fig.1 B.

L'affinité pour le récepteur α_{2A} -adrénergique a été étudiée (voir tableau I au point 2.2). Aucun déplacement significatif n'a été observé à la concentration de 10⁻⁵ M.

Les affinités du composé 1 pour plus de 50 récepteurs et transporteurs ont été déterminées. Le composé 1 ne présente aucune affinité similaire à celle pour le récepteur des imidazolines I₁ (figure 3).

### 1.4 Production d'adiponectine

### 1.4.1. Mode opératoire

Pour déterminer la sécrétion d'adiponectine par les adipocytes matures, des adipocytes différentiés 3T3-L1 (10 jours après confluence) sont mis en culture dans des plaques 12 puits et lavés trois fois avec du DMEM, puis cultivés pendant 6 h dans le DMEM seul, en l'absence ou en présence de 3 µM du composé 1. Dans certains puits, l'antagoniste sélectif des récepteurs des imidazolines I₁, efaroxan (100 µM) a été ajouté 30 min avant l'exposition au composé 1.

Le milieu de culture a été ensuite collecté, centrifugé pendant 3 min à 10 000g pour retirer les contaminants cellulaires, et le surnageant a été stocké à -80°C jusqu'à utilisation. Après deux lavages par du PBS froid, les adipocytes ont été collectés dans du PBS, homogénéisés, et stockés à -80°C. Une aliquote d'homogénat d'adipocyte a été conservée pour la détermination des protéines. L'adiponectine a été mesurée par un kit ELISA selon les recommandations du fournisseur. La concentration en adiponectine a été normalisée avec le contenu protéique cellulaire.

### 1.4.2. Résultats

Les résultats sont présentés figure 2.

Le composé 1 à une dose de 3 µmol/l a induit une augmentation de la sécrétion d'adiponectine par les adipocytes 3T3-L1 (378 ± 38 vs 212,6 ± 19,1 ng/ml/mg de protéines p<0,001). Une pré incubation avec de l'efaroxan, un antagoniste sélectif des récepteurs I₁ à une dose de 100 µmol/l, antagonise l'augmentation de la sécrétion d'adiponectine (236 ± 19,2 vs 378 ± 38 ng/ml/mg de protéines, p<0,001) tandis que l'efaroxan seul, à la même dose, n'a pas d'effet significatif comparé au témoin (175,2 ± 15,5 vs 212,6 ± 19,1 ng/ml/mg de protéines, p>0,05).

### EXEMPLE 2 : Etude de liaison spécifique sur les plaquettes humaines.

### 2.1 Mode opératoire

### 2.1.1. Etude de liaison spécifique du récepteur I₁.

Les tests de liaison ont été effectués à 37 °C utilisant comme radioligand le [¹²⁵I]LNP 911 selon la procédure générale décrite mais adaptée aux plaquettes entières lavées (Greney, H,; Urosevic, D,; Schann, S,; Dupuy, L,; Bruban, V,; Ehrhardt, J,-D,; Bousquet, P,; Dontenwill, M. [125I]2-(2-Chloro-4-iodo-phénylamino)-5-méthyl-pyrroline (LNP911), a High-Affinity Radioligand Selective for I1 Imidazoline Receptors. Mol. Pharmaco/. 2002, 62, 181-191).

L'incubation a été initiée par addition de 900 à 950 µl de suspension de plaquettes à une concentration de 500000/µl dans un volume final de 1 ml de Tyrode albumine et a été effectuée à 37 °C durant 5 min (conditions d'équilibre). Les essais de compétition ont été effectués en utilisant une concentration unique de radioligand (50 pM, 200 000 cpm), en présence de concentration croissante de ligand non marqué approprié. La liaison non spécifique a été déterminée par la liaison de [¹²⁵I] LNP 911 en présence de 100 nM de LNP 911 non marqué et représente environ 10% de la radioactivité totale quand 50 pM de [¹²⁵I] LNP 911 sont utilisés.

La réaction a été arrêtée par filtration rapide sous vide à travers des filtres en fibre de verre GF/C suivi par cinq lavages rapides des filtres avec 3 ml de Tyrode glacé (137 nM NaCl, 2,7 nM KCl, 12 nM NaHCO₃, 0,36 nM NaH₂PO₄, pH 7,35). La radioactivité a été mesurée sur un compteur gamma (Wallac 1410).

### 2.1.2 Etude de liaison des récepteurs α₂ adrénergiques.

La préparation membranaire a été réalisée comme décrit par Newman-Tancredi, A,; Nicolas, J,-P,; Audinot, V,; Gavaudan, S,; Verriele, L,; Touzard, M,; Chaput, C,; Richard, N,; Millan, N. J. (Action of alpha2 Adrenoreceptor Ligands at alpha2A and 5-HT1A Receptors: the Antagonist, Atipamezole, and the Agonist, Dexmedetomidine, are Highly Selective for alpha2A Adrenorecptors. Naunyn-Schmiedeberg's Arch. Pharmacol. 1998, 358, 197-206).

Ces membranes (30 µg protéines/ml pour CHO-hα_{2A} et CHO-hα_{2B}, 100 µg protéine /ml pour CHO-hα_{2C}) ont été incubées pendant 60 min à température ambiante dans un tampon de liaison (33 mM Tris HCl, 1 mM EDTA, pH 7,5) dans un volume final de 500 µL contenant 0,8 ou 1 ou 2 nM [³H]RX821002 respectivement pour les récepteurs adrénergiques hα_{2A}, hα_{2B} et hα_{2C}.

L'incubation a été arrêtée par filtration rapide sous vide à travers des filtres en fibre de verre GF/C suivi par trois lavages successifs avec un tampon de liaison glacé.

La liaison non spécifique a été déterminée par 10 µM de phentolamine.

### 2.2 Résultats

Les essais de compétition ont été analysés par régression non linéaire en utilisant le programme GraphPad

Les *K*i ont été déterminés en utilisant la méthode de Cheng et Prussof (Cheng, Y. C,; Prusoff, W. H. Relationship between the Inhibition Constant (Ki) and the Concentration of Inhibitor which Causes 50 per Cent Inhibition (I50) of an Enzymatic Reaction. Biochem. Pharmacol. 1973, 22,3099-3108).
Les résultats sont présentés dans le tableau I ci-dessous :

**TABLEAU I**

| Composés | I₁ *Ki* (M)# | α₂ *Ki* (M)^{§} |
|---|---|---|
| 1 | 8,1 × 10⁻⁹ | > 10⁻⁵ |
| 5 | 1,9 × 10⁻⁸ | > 10⁻⁵ |
| 8 | 2,2 × 10⁻¹⁰ | > 10⁻⁵ |
| 9 | 2,8 × 10⁻⁹ | > 10⁻⁵ |
| 13 (comparatif) | 1,2 × 10⁻⁷ | > 10⁻⁵ |
| 20 | 2,8 × 10⁻⁹ | > 10⁻⁵ |

| | | |
|---|---|---|
| # mesuré sur plaquettes avec [¹²⁵I]LNP 911, sauf pour le composé 13 dont la mesure est effectuée sur des cellules PC12 avec [¹²⁵I]PIC; § mesuré sur des cellules CHO avec de la phentolamine. | | |

Les résultats présentés ci-dessus montrent clairement la spécificité des composés de l'invention pour le récepteur aux imidazolines de type 1 par rapport aux récepteurs adrénergiques α₂.

### EXEMPLE 3: Etude de liaison spécifique du composé 1 sur différents récepteurs, transporteurs et enzymes

### 3.1. Mode opératoire

Le composé 1 a été testé à deux concentrations 10⁻⁷ (1.0E-07) M et 10⁻⁵ M (1.0E-05) sur différents récepteurs, transporteurs et enzymes selon des techniques décrites dans la littérature et adaptées au laboratoire.

### 3.2. Résultats

Ils sont donnés dans le tableau de la figure 3.

Les résultats présentés ci-dessus montrent clairement que le composé 1 ne se lie à aucun des récepteurs, transporteurs et enzymes testés avec une affinité similaire à celle qu'a le composé 1 pour les RI₁.

### EXEMPLE 4 : Mesure de l'activité rénale nerveuse sympathique (RSNA) : Effet d'un traitement aigu par le composé 1 à 10 mg/kg (i.v.) sur les paramètres hémodynamiques et l'activité sympathique.

### 4.1. Mode opératoire

Des rats mâles Sprague-Dawley (10 semaines; Laboratoires Charles River, L'Arbresle, France) ont été anesthésiés avec de l'uréthane (1,5 g/kg i.p., complété par 0,1 g/kg i.v. si nécessaire) et placés sur une couverture chauffante pour maintenir la température rectale à 37°C. Des cathéters ont été insérés dans l'aorte abdominale inférieure et la veine cave inférieure pour la mesure de la pression artérielle et l'administration du composé respectivement.

Le nerf rénal gauche a été isolé soigneusement et une branche majeure du nerf a été placée sur une électrode bipolaire platine-iridium et isolée avec du gel de silicone (604A et B; Wacker Chemie, Munich, Allemagne). Tout le long de l'expérience, le rat a été ventilé au moyen d'une canule trachéale (7-8 ml/kg X 72 cycles/min) avec un mélange d'oxygène et d'air (~8%-20%).

La pression artérielle a été mesurée en connectant le cathéter artériel à un transducteur de pression (TNF-R; Ohmeda, Bilthoven, Hollande) couplé à un amplificateur (model 13-4615-52; Gould, Cleveland, OH).

L'activité du nerf sympathique rénal (ANSR) a été amplifiée (X50,000), la bande passante filtrée (300-3 000 Hz: Model P-511J; Grass, Quincy, MA), et rectifiée par un rectifieur analogique fait maison incluant un filtre basse fréquence avec une fréquence de coupure de 150 Hz.

Les 2 paramètres ont été enregistrés en continu grâce à un ordinateur équipé d'un convertisseur analogique digital (model AT-MIO-16; National Instruments, Austin, TX), et logiciel LabVIEW 5,1 (National Instruments).

La pression artérielle et l'ANSR ont été enregistrées avant (ligne de base) et après administration de composé 1 (10 mg/kg, i.v.). A la fin de la session d'enregistrement, la chlorisondamine, bloqueur ganglionnaire, a été administrée (2,5 mg/kg i.v.) pour évaluer le niveau de bruit de fond qui a été ensuite soustrait de toutes les données de l'ANSR pour les analyses suivantes. A la fin de l'expérience, les rats ont été euthanasiés avec une overdose intraveineuse de pentobarbital sodique.

### 4.2. Résultats

Les résultats sont présentés figures 4A à 4C.

Le composé 1, à une dose de 10 mg/kg, iv, provoque une diminution de 50% de l'activité sympathique rénale (figure 4A).

En parallèle, le compose 1 à la même concentration provoque une forte diminution de la pression artérielle (PAS: 105,3±3 vs 141,7±2,3 mmHg, p<0,001; PAM: 77,4±3,6 vs 108±3,4 mmHg, p<0,0001; PAD: 58,6±3,4 vs 83,2±4 mmHg, p<0,001) et de la fréquence cardiaque (288,4±10,3 vs 331,4±14 bpm, p<0,01) (figures 4B et 4C). La diminution de l'activité sympathique rénale, de la pression artérielle et de la fréquence cardiaque dure plus d'une heure.

### EXEMPLE 5 : Mesure de la pression artérielle, de la fréquence cardiaque, des paramètres biochimiques plasmatiques et le métabolisme du glucose : Effet d'un traitement chronique par le composé 1 à la dose de 20 mg/kg/j per os pendant 12 semaines

### 5.1. Mode opératoire

### 5.1.1 Animaux

Des rats mâles SHHF (*spontaneously hypertensive*, *heart failure* ; *spontanément hypertendu*, *insuffisance cardiaque*) âgés de 12 semaines ont été utilisés dans cette étude (Centre d'Elevage Charles River, L'Arbresle, France).

Les animaux ont été placés dans une pièce à température et lumière contrôlées avec un accès libre à l'eau du robinet et ont été nourris avec un régime standard (A04, SAFE, Augy France). Cette étude a été menée en accord avec les recommandations institutionnelles et les règles formulées par la Communauté Européenne pour l'utilisation d'animaux expérimentaux (L358-86/609/EEC).

### 5.1.2 Traitement chronique par le composé 1

Le composé 1 a été administré dans l'eau de boisson pendant 12 semaines à la dose de 20 mg/kg/j (n=17). La consommation d'eau est mesurée régulièrement de manière à ajuster la concentration en composé 1. Des rats SHHF témoins non traités ont bu de l'eau normale (n=10).

Le poids corporel, la prise d'eau et de nourriture ont été mesurés tous les jours. Après 12 semaines de traitement, des prélèvements sanguins ont été effectués. Trois jours après, la pression artérielle et la fréquence cardiaque ont été enregistrées et un test de tolérance au glucose a été effectué.

### 5.1.3. Enregistrement de la pression artérielle et de la fréquence cardiaque

Les rats ont été anesthésiés avec du pentobarbital 50 mg/kg, ip (Céva santé animale, Libourne, France) et trachéotomisés. La veine et l'artère fémorale ont été cathétérisées pour l'administration des substances et la mesure de la pression artérielle respectivement. Les rats ont été ventilés avec l'air ambiant et paralysés par une administration de bromure de pancuronium (1,5 mg/kg, iv ; Organon SA, France). La pression artérielle a été enregistrée après stabilisation avec un transducteur de pression (Gould P23XL) et un enregistreur (Gould electronics BS 272, Longjumeau, France). La pression artérielle moyenne (MAP) a été calculée comme la pression diastolique plus un tiers de la pression artérielle différentielle. La fréquence cardiaque a été enregistrée également à partir du signal de pression avec un amplificateur Gould Biotach (model 13-4615-66).

Les résultats sont présentés dans les figures 6A et 6B.

Après 12 semaines de traitement à la dose de 20 mg/kg/j par le composé 1 dans l'eau de boisson, la pression artérielle des rats SHHF est significativement diminuée (153±7 vs 176±6 mmHg, p<0,05) (figure 6A), mais la fréquence cardiaque n'est pas modifiée (367±6 vs 361±8 bpm, p>0,05) (figure 6B).

### 5.1.4 Mesure des paramètres biochimiques plasmatiques

Après 12 semaines de traitement, des échantillons sanguins ont été obtenus à partir de veine caudale de la queue de rats anesthésiés (isoflurane 2,5%, Abbott, Rungis, France) après un jeûne de 18h.

Les échantillons sanguins ont été centrifugés pendant 15 minutes à 2000 g et le plasma a été congelé à -80°C jusqu'aux dosages du glucose, du cholestérol total, des cholestérols HDL et LDL, et des acides gras. Ces dosages ont été effectués sur le "Plateau Technique Biologique des Hôpitaux Universitaires de Strasbourg" (Advia 2400, Bayer HealthCare).

L'insuline, la leptine, l'adiponectine et le glucagon ont été dosés par des kits ELISA (insuline: Mercodia, Uppsala, Suède, leptine: Yanaihara Institute Inc" Shizuoka, Japon adiponectine: B-Bridge International, Mountain View, USA et glucagon: Gentaur, Kampenhout, Belgique) selon les recommandations du fournisseur.

Les résultats sont présentés figures 7A à 7C et figures 8A à 8E.

Après 12 semaines de traitement avec le composé 1, une importante diminution du cholesterol plasmatique total est observée (2,7±0,09 vs 3,8±0,18 mmol/l, P< 0,0001) (figure 7A). Le composé 1 provoque aussi une baisse de la concentration en triglycérides plasmatiques (3,8±0,25 vs 4,59±0,23 mmol/l, p<0,01) (figure 7B) ; les acides gras libres ne sont pas affectés par le traitement (figure 7C).

La glycémie à jeun n'est pas affectée par le traitement (figure 8A), mais l'insuline plasmatique à jeun est diminuée significativement (15,2±2 vs 46,8±3,7 ng/ml, p<0,0001) (figure 8B).

Le calcul de l'index de HOMA-IR confirme que les rats SHHF traités ont une meilleure sensibilité à l'insuline que les rats SHHF témoins (110±14 vs 534±38, p<0,0001) (figure 8C). Enfin, le composé 1 provoque une baisse significative de glucagon (77,3±14 vs 161,3±33 pg/ml, p<0,05) (figure 6D) et une forte augmentation de la concentration en adiponectine plasmatique (10,6±0,52 vs 5,54±0,14 µg/l, p<0,000l) (figure 8E).

### 5.1.5 Test de tolérance au glucose (IVGTT)

Une solution de glucose à 0,5 g/kg a été administrée par voie intra veineuse. La concentration plasmatique de glucose a été évaluée en utilisant un glycomètre avant l'injection aux animaux à jeun depuis 18h, puis 3, 6, 10, 15, 30, et 45 min après l'administration du glucose (Accu Check Go, Roche Diagnostics, Meylan, France). Les aires sous les courbes (AUC) ont ensuite été déterminées pour comparer les groupes.

### 5.2. Résultats

### 5.2.1 Mesure du poids corporel et la consommation de nourriture

Les résultats sur le poids et la consommation de nourriture sont présentés figures 5A et 5B.

Après 9 semaines de traitement et jusqu'à la fin du traitement, les rats SHHF traités par le composé 1 présentent un poids corporel inférieur à celui des témoins ; leur consommation de nourriture est déjà inférieure à celle des témoins après une semaine de traitement (figures 5A et 5B).

### 5.2.2. Mesure des paramètres biochimiques plasmatiques

Les résultats sont présentés figures 7A à 7C et figures 8A à 8E.

Après 12 semaines de traitement avec le composé 1, une importante diminution du cholesterol plasmatique total est observée (2,7±0,09 vs 3,8±0,18 mmol/l, P< 0,0001) (figure 7A). Le composé 1 provoque aussi une baisse de la concentration en triglycérides plasmatiques (3,8±0,25 vs 4,59±0,23 mmol/l, p<0,01) (figure 7B) ; les acides gras libres ne sont pas affectés par le traitement (figure 7C).

La glycémie à jeun n'est pas affectée par le traitement (figure 8A), mais l'insuline plasmatique à jeun est diminuée significativement (15,2±2 vs 46,8±3,7 ng/ml, p<0,0001) (figure 8B).

Le calcul de l'index de HOMA-IR confirme que les rats SHHF traités ont une meilleure sensibilité à l'insuline que les rats SHHF témoins (110±14 vs 534±38, p<0,0001) (figure 8C). Enfin, le composé 1 provoque une baisse significative de glucagon (77,3±14 vs 161,3±33 pg/ml, p<0,05) (figure 6D) et une forte augmentation de la concentration en adiponectine plasmatique (10,6±0,52 vs 5,54±0,14 µg/l, p<0,000l) (figure 8E).

### 5.2.3. Test de tolérance au glucose (IVGTT)

Les résultats sont présentés figures 8F et 8G.

Le composé 1 augmente la tolérance au glucose des rats SHHF (AUC : 556±20 vs 710±37 mmol.min/l, p<0,001) (figures 8F et 8G).

### EXEMPLE 6 : Mesure de la pression artérielle et de la fréquence cardiaque : Effet d'un traitement aigu par voie intraveineuse ou intracisternale

### 6.1. Mode opératoire

Des rats mâles adultes Wistar ont été anesthésiés avec du pentobarbital 50 mg/kg, ip et leur température physiologique a été maintenue constante. Les animaux ont été trachéotomisés et la carotide gauche a été cathérisée pour permettre l'administration i.v. Les animaux ont été paralysés par une administration de bromure de pancuronium (1 mg/kg, iv ; Organon SA, France) et ventilés avec l'air ambiant (Hugo Sachs modèle électronique 7025). Un cathéter a été introduit dans l'artère fémorale gauche et connecté à un transducteur de pression et un enregistreur (Gould electronics BS 272, Longjumeau, France). La fréquence cardiaque, la pression artérielle systolique, la pression artérielle diastolique et la pression artérielle moyenne (PAM) ont été enregistrées en continu à partir du signal de pression (Gould Biotach amplifier model 13-4615-66) ; [PAM = PAD +1/3 (PAS-PAD)]. La pression artérielle moyenne (PAM) et la fréquence cardiaque (FC) sont mesurées pendant 90 minutes.

### 6.2. Résultats

Les résultats sont exprimés comme la variation maximale de la pression artérielle moyenne exprimée en mm de mercure (mmHg) et de la fréquence cardiaque exprimée en battements par minutes (bpm) comparativement aux valeurs basales avant traitement. Le pourcentage de variation correspondant est également déterminé. Les résultats sont considérés comme significatifs lorsque la variation est supérieure de 10 % à la valer de base.

Dans les expériences où la drogue est injectée par la voie intracisternale, 0,2 ml d'une solution de drogue est injecté après retrait d'un volume identique de liquide céphalorachidien.

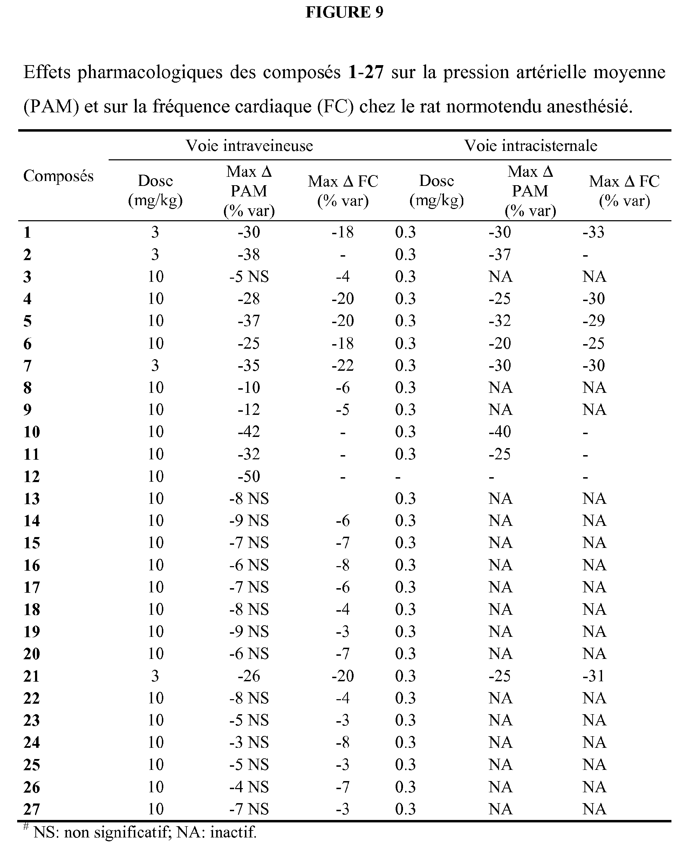

Les résultats sont présentés dans le tableau de la Figure 9.

## Revendications

1. Composés de formule générale (I) suivante : 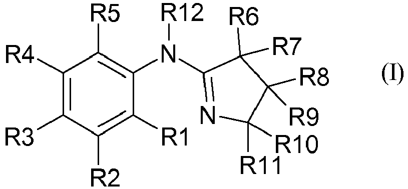 dans laquelle :
a) soit R12 représente H, et
- R1 et R2 représentent indépendamment les uns des autres :
un halogène, un alkyle en C1 à C8 linéaire ou ramifié, un alcène en C2-C8, un alcoxy en C1 à C8 linéaire ou ramifié, un cycloalkyle en C3-C6, un bicycloalkyle en C5-C6, une chaîne polyéther, un perfluoroalkyle en C1-C5, un acyle en C1-C8, OH, SH, une amine primaire, secondaire ou tertiaire, CN, CO₂H, CO₂R' dans lequel R' est un alkyle linéaire ou ramifié en C1-C8, ou un cycloalkyle en C3-C6,
ou
R1 et R2 forment ensemble un cycle en C5,
- R3, R4 et R5 représentent indépendamment les uns des autres :
un hydrogène, un halogène, un alkyle en C1 à C8 linéaire ou ramifié, un alcène en C2-C8, un alcoxy en C1 à C8 linéaire ou ramifié, un cycloalkyle en C3-C6, un bicycloalkyle en C5-C6, une chaîne polyéther, un perfluoroalkyle en C1-C5, un acyle en C1-C8, OH, SH, une amine primaire, secondaire ou tertiaire, CN, CO₂H, CO₂R' dans lequel R' est un alkyle linéaire ou ramifié en C1-C8, ou un cycloalkyle en C3-C6,
- R6, R7, R9 et R11 représentent des hydrogènes,
- R8 et R10 sont sélectionnés parmi le groupe consistant en un hydrogène et un alkyle en C1 à C5 linéaire ou ramifié, au moins l'un des deux étant un alkyle en C1 à C5 linéaire ou ramifié ;
b) soit R12 représente CH(R13)(CH₂) et forme un cycle en C5 avec R5, R13 représentant H ou CH₃,
- R1, R2, R3 et R4 sont sélectionnés parmi le groupe consistant en un hydrogène, un halogène, un alkyle en C1 à C8 linéaire ou ramifié, un alcène en C2-C8, un alcoxy en C1 à C8 linéaire ou ramifié, un cycloalkyle en C3-C6, un bicycloalkyle en C5-C6, une chaîne polyéther, un perfluoroalkyle en C1-C5, un acyle en C1-C8, -OH, -SH, une amine primaire, secondaire ou tertiaire, -CN, -CO₂H, -CO₂R' dans lequel R' est un alkyle linéaire ou ramifié en C1-C8, et un cycloalkyle en C3-C6 ;
- R6, R7, R9 et R11 représentent des hydrogènes,
- R8 et R10 sont sélectionnés parmi le groupe consistant en un hydrogène et un alkyle en C1 à C5 linéaire ou ramifié, au moins l'un des deux étant un alkyle en C1 à C5 linéaire ou ramifié,
à condition qu'au moins un parmi R1 et R13 ne soit pas un hydrogène,
et leurs sels pharmacologiquement acceptables.

2. Composés selon la revendication 1, de formule générale (Ia) suivante : 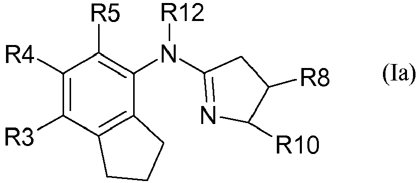 dans laquelle
R3 et R12 sont des hydrogènes,
R8 et R10 sont sélectionnés parmi le groupe consistant en un hydrogène et un alkyle en C1 à C5 linéaire ou ramifié, au moins l'un des deux étant un alkyle en C1 à C5 linéaire ou ramifié, et
R4 et R5 sont sélectionnés indépendamment parmi le groupe consistant en un hydrogène, un halogène, un alkyle en C1 à C3 linéaire ou ramifié, un alcoxy en C1 à C3 linéaire ou ramifié, un perfluoroalkyle en C1-C3, un acyle en C1-C3, OH, SH, une amine primaire, secondaire ou tertiaire, CN, CO₂H, et CO₂R' dans lequel R' est un alkyle linéaire ou ramifié en C1-C3, de préférence parmi groupe consistant en un halogène, un alkyle en C1 à C3 linéaire ou ramifié, un alcoxy en C1 à C3 linéaire ou ramifié, un perfluoroalkyle en C1-C3, et un acyle en C1-C3.

3. Composés selon la revendication 2, dans laquelle R4 et R5 sont des hydrogènes.

4. Composés selon la revendication 1, de formule générale (Ib) suivante : 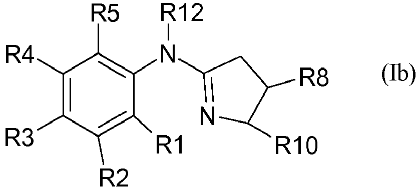 dans laquelle
R12 est un hydrogène,
R8 et R10 sont sélectionnés parmi le groupe consistant en un hydrogène et un alkyle en C1 à C5 linéaire ou ramifié, au moins l'un des deux étant un alkyle en C1 à C5 linéaire ou ramifié,
R1 et R2 sont sélectionnés indépendamment parmi le groupe consistant en un halogène, un alkyle en C1 à C3 linéaire ou ramifié, un alcoxy en C1 à C3 linéaire ou ramifié, un perfluoroalkyle en C1-C3, et un acyle en C1-C3, et
R3, R4 et R5 sont sélectionnés indépendamment parmi le groupe consistant en un hydrogène, un halogène, un alkyle en C1 à C3 linéaire ou ramifié, un alcoxy en C1 à C3 linéaire ou ramifié, un perfluoroalkyle en C1-C3, un acyle en C1-C3, OH, SH, une amine primaire, secondaire ou tertiaire, CN, CO₂H, et CO₂R' dans lequel R' est un alkyle linéaire ou ramifié en C1-C3, de préférence parmi groupe consistant en un halogène, un alkyle en C1 à C3 linéaire ou ramifié, un alcoxy en C1 à C3 linéaire ou ramifié, un perfluoroalkyle en C1-C3, et un acyle en C1-C3.

5. Composés selon la revendication 4, dans laquelle R3, R4 et R5 sont des hydrogènes.

6. Composés selon la revendication 4 ou 5, dans laquelle R1 et R2 sont sélectionnés indépendamment parmi le groupe consistant en un halogène et un alkyle en C1 à C3 linéaire ou ramifié.

7. Composé selon l'une des revendications 1 à 6, choisis parmi l'une des formules suivantes:

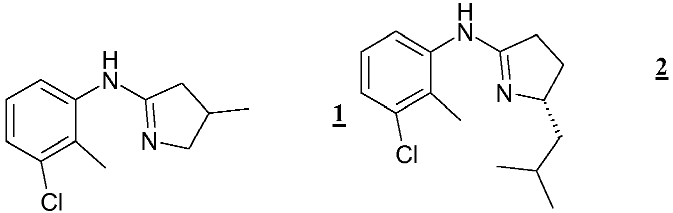

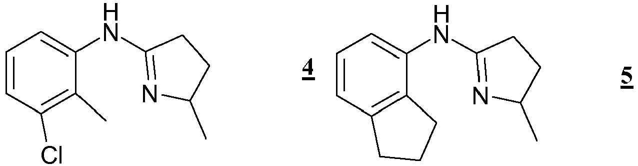

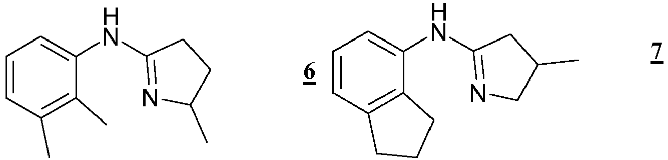

8. Composés selon la revendication 1, de formule générale (Ic) suivante : 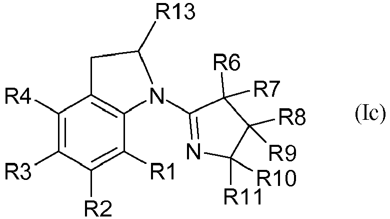 dans laquelle,
- R1, R2, R3 et R4 sont sélectionnés parmi le groupe consistant en un hydrogène, un halogène, un alkyle en C1 à C3 linéaire ou ramifié, un alcoxy en C1 à C3 linéaire ou ramifié, un perfluoroalkyle en C1-C3, un acyle en C1-C3, OH, SH, une amine primaire, secondaire ou tertiaire, -CN, -CO₂H, et -CO₂R' dans lequel R' est un alkyle linéaire ou ramifié en C1-C3;
- R8 et R10 sont sélectionnés parmi le groupe consistant en un hydrogène et un alkyle en C1 à C5 linéaire ou ramifié, au moins l'un des deux étant un hydrogène,
à condition qu'au moins un parmi R1 et R13 ne soit pas un hydrogène.

9. Composés selon la revendication 8, dans laquelle
- R2, R3, R4 sont des hydrogènes,
- R13 est H et R1 est un halogène et un alkyle en C1 à C3 linéaire ou ramifié; ou
R13 est un méthyle, et R1 est sélectionné parmi le groupe consistant en un hydrogène, un halogène et un alkyle en C1 à C3 linéaire ou ramifié ; et
- R8 et R10 sont sélectionnés parmi le groupe consistant en un hydrogène et un alkyle en C1 à C5 linéaire ou ramifié, au moins l'un des deux étant un hydrogène.

10. Composés selon la revendication 8 ou 9, choisis parmi l'une des formules suivantes:

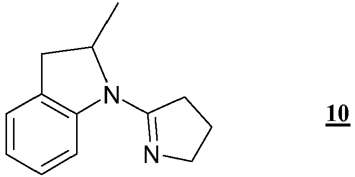

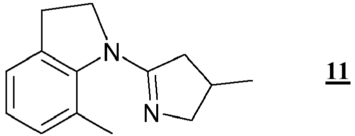

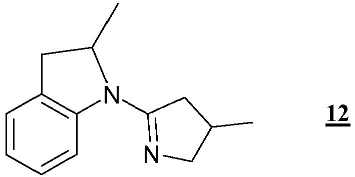

11. Composés tels que définis dans l'une des revendications 1 à 10, pour leur utilisation pour la prévention et/ou le traitement du syndrome métabolique.

12. Composition pharmaceutique comprenant comme principe actif au moins un composé selon l'une des revendications 1 à 10 en association avec un véhicule pharmaceutiquement acceptable.

13. Composition pharmaceutique selon la revendication 12, administrable par voie orale.

14. Composition pharmaceutique administrable par voie orale selon la revendication 13, dans laquelle le principe actif est à une dose comprise de 1 mg/kg à 100 mg/kg chez l'homme.

## Patentansprüche

1. Verbindungen der folgenden allgemeinen Formel (I): 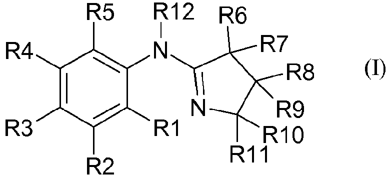 in der:
a) entweder R12 für H steht, und
- R1 und R2 unabhängig voneinander stehen für:
ein Halogen, ein verzweigtes oder lineares C1 bis C8-Alkyl, ein C2-C8-Alken, ein verzweigtes oder lineares C1 bis C8-Alkoxy, ein C3-C6-Cycloalkyl, ein C5-C6-Bicycloalkyl, eine Polyetherkette, ein C1-C5-Perfluoralkyl, ein C1-C8-Acyl, OH, SH, ein primäres, sekundäres oder tertiäres Amin, CN, CO₂H, CO₂R', in dem R' ein verzweigtes oder lineares C1 bis C8-Alkyl oder ein C3-C6-Cycloalkyl ist, oder
- R1 und R2 miteinander einen C5-Ring bilden;
- R3, R4 und R5 unabhängig voneinander stehen für:
einen Wasserstoff, ein Halogen, ein verzweigtes oder lineares C1 bis C8-Alkyl, ein C2-C8-Alken, ein verzweigtes oder lineares C1 bis C8-Alkoxy, ein C3-C6-Cycloalkyl, ein C5-C6-Bicycloalkyl, eine Polyetherkette, ein C1-C5-Perfluoralkyl, ein C1-C8-Acyl, OH, SH, ein primäres, sekundäres oder tertiäres Amin, CN, CO₂H, CO₂R', in dem R' ein verzweigtes oder lineares C1 bis C8-Alkyl oder ein C3-C6-Cycloalkyl ist,
- R6, R7, R9 und R11 für Wasserstoff stehen,
- R8 und R10 aus der Gruppe gewählt sind, bestehend aus einem Wasserstoff und einem verzweigten oder linearen C1 bis C5-Alkyl, wobei wenigstens einer der beiden Reste ein verzweigtes oder lineares C1 bis C5-Alkyl ist;
b) oder R12 für CH(R13)(CH₂) steht und einen C5-Ring mit R5 bildet, wobei R13 für H oder CH₃ steht,
- R1, R2, R3 und R4 aus der Gruppe ausgewählt sind, bestehend aus einem Wasserstoff, einem Halogen, einem verzweigten oder linearen C1 bis C8-Alkyl, einem C2-C8-Alken, einem verzweigten oder linearen C1 bis C8-Alkoxy, einem C3-C6-Cycloalkyl, einem C5-C6-Bicycloalkyl, einer Polyetherkette, einem C1-C5-Perfluoralkyl, einem C1-C8-Acyl, - OH, -SH, einem primären, sekundären oder tertiären Amin, -CN, -CO₂H, -CO₂R', in dem R' ein verzweigtes oder lineares C1 bis C8-Alkyl oder ein C3-C6-Cycloalkyl ist,
- R6, R7, R9 und R11 für Wasserstoff stehen,
- R8 und R10 aus der Gruppe gewählt sind, bestehend aus einem Wasserstoff und einem verzweigten oder linearen C1 bis C5-Alkyl, wobei wenigstens einer der beiden Reste ein verzweigtes oder lineares C1 bis C5-Alkyl ist;
unter der Bedingung, dass wenigstens einer der Reste R1 und R13 kein Wasserstoff ist,
und ihre pharmakologisch verträglichen Salze.

2. Verbindungen gemäß Anspruch 1 der folgenden allgemeinen Formel (Ia): 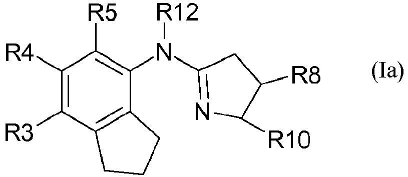 in der
R3 und R12 Wasserstoff sind,
R8 und R10 aus der Gruppe ausgewählt sind, bestehend aus einem Wasserstoff und einem verzweigten oder linearen C1 bis C5-Alkyl, wobei wenigstens einer der beiden Reste ein verzweigtes oder lineares C1 bis C5-Alkyl ist, und
R4 und R5 unabhängig voneinander aus der Gruppe, bestehend aus einem Wasserstoff, einem Halogen, einem verzweigten oder linearen C1 bis C3-Alkyl, einem verzweigten oder linearen C1 bis C3-Alkoxy, einem C1-C3-Perfluoralkyl, einem C1-C3-Acyl, OH, SH, einem primären, sekundären oder tertiären Amin, CN, CO₂H, und CO₂R', in dem R' ein verzweigtes oder lineares C1 bis C3-Alkyl ist, vorzugsweise aus der Gruppe ausgewählt sind, bestehend aus einem Halogen, einem linearen oder verzweigten C1 bis C3-Alkyl, einem verzweigten oder linearen C1-C3-Alkoxy, einem C1-C3-Perfluoralkyl und einem C1-C3-Acyl.

3. Verbindungen gemäß Anspruch 2, wobei R4 und R5 Wasserstoff sind.

4. Verbindungen gemäß Anspruch 1 der folgenden allgemeinen Formel (Ib): 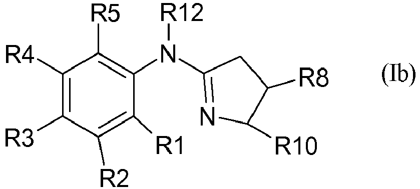 in der
R12 Wasserstoff ist,
R8 und R10 aus der Gruppe ausgewählt sind, bestehend aus einem Wasserstoff und einem verzweigten oder linearen C1 bis C5-Alkyl, wobei wenigstens einer der beiden Reste ein verzweigtes oder lineares C1-C5-Alkyl ist,
R1 und R2 unabhängig voneinander aus der Gruppe ausgewählt sind, bestehend aus einem Halogen, einem verzweigten oder linearen C1 bis C3-Alkyl, einem verzweigten oder linearen C1 bis C3-Alkoxy, einem C1-C3-Perfluoralkyl und einem C1-C3-Acyl, und
R3, R4 und R5 unabhängig voneinander aus der Gruppe, bestehend aus einem Wasserstoff, einem Halogen, einem verzweigten oder linearen C1 bis C3-Alkyl, einem verzweigten oder linearen C1 bis C3-Alkoxy, einem C1-C3-Perfluoralkyl, einem C1-C3-Acyl, OH, SH, einem primären, sekundären oder tertiären Amin, CN, CO₂H, CO₂R', in dem R' ein verzweigtes oder lineares C1 bis C3-Alkyl ist, vorzugsweise aus der Gruppe ausgewählt sind, bestehend aus einem Halogen, einem verzweigten oder linearen C1 bis C3-Alkyl, einem verzweigten oder linearen C1 bis C3-Alkoxy, einem C1-C3-Perfluoralkyl und einem C1-C3-Acyl.

5. Verbindungen gemäß Anspruch 4, wobei R3, R4 und R5 Wasserstoff sind.

6. Verbindungen gemäß Anspruch 4 oder 5, wobei R1 und R2 unabhängig voneinander aus der Gruppe ausgewählt sind, bestehend aus einem Halogen und einem verzweigten oder linearen C1 bis C3-Alkyl.

7. Verbindung gemäß einem der Ansprüche 1 bis 6, die aus einer der folgenden Formeln ausgewählt ist:

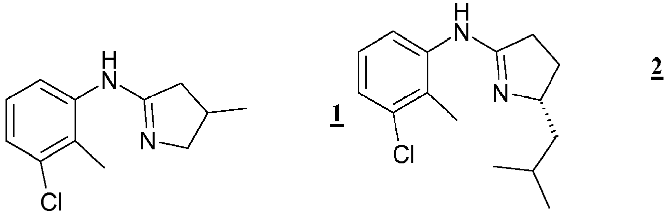

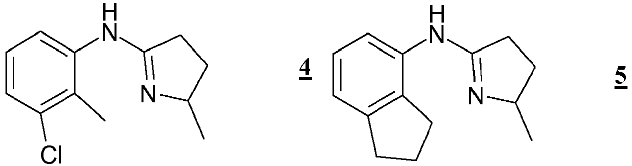

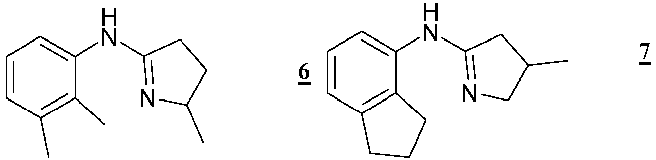

8. Verbindungen gemäß Anspruch 1 der folgenden allgemeinen Formel (Ic): 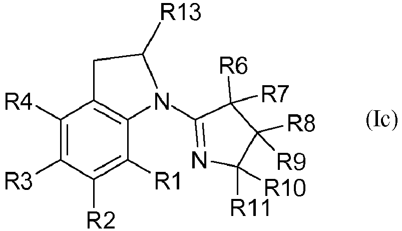 in der
- R1, R2, R3 und R4 aus der Gruppe ausgewählt sind, bestehend aus einem Wasserstoff, einem Halogen, einem verzweigten oder linearen C1 bis C3-Alkyl, einem verzweigten oder linearen C1 bis C3-Alkoxy, einem C1-C3-Perfluoralkyl, einem C1-C3-Acyl, OH, SH, einem primären, sekundären oder tertiären Amin, -CN, -CO₂H und -CO₂R', in dem R' ein verzweigtes oder lineares C1 bis C3-Alkyl ist;
- R8 und R10 aus der Gruppe ausgewählt sind, bestehend aus einem Wasserstoff und einem verzweigten oder linearen C1 bis C5-Alkyl, wobei wenigstens einer der beiden Reste ein Wasserstoff ist,
unter der Bedingung, dass wenigstens einer der Reste R1 und R13 kein Wasserstoff ist.

9. Verbindungen gemäß Anspruch 8, wobei
- R2, R3, R4 Wasserstoff sind,
- R13 H ist und R1 ein Halogen und ein verzweigtes oder lineares C1 bis C3-Alkyl ist; oder
- R13 ein Methyl ist und R1 aus der Gruppe ausgewählt ist, bestehend aus einem Wasserstoff, einem Halogen und einem verzweigten oder linearen C1 bis C3-Alkyl; und
- R8 und R10 aus der Gruppe ausgewählt sind, bestehend aus einem Wasserstoff und einem verzweigten oder linearen C1 bis C5-Alkyl, wobei wenigstens einer der beiden Reste ein Wasserstoff ist.

10. Verbindungen gemäß Anspruch 8 oder 9, die aus einer der folgenden Formeln ausgewählt sind:

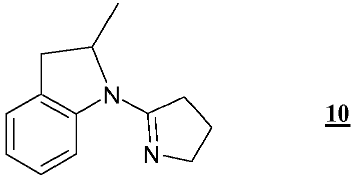

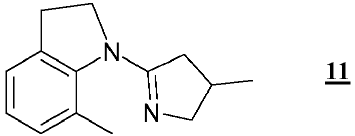

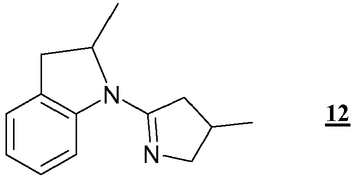

11. Verbindungen, wie in einem der Ansprüche 1 bis 10 definiert, für ihre Verwendung für die Prävention und/oder Behandlung des metabolischen Syndroms.

12. Pharmazeutische Zusammensetzung, umfassend als Wirkstoff wenigstens eine Verbindung gemäß einem der Ansprüche 1 bis 10 in Kombination mit einem pharmazeutisch verträglichen Vehikel.

13. Pharmazeutische Zusammensetzung gemäß Anspruch 12 zur oralen Verabreichung.

14. Pharmazeutische Zusammensetzung zur oralen Verabreichung gemäß Anspruch 13, wobei die Wirkstoff-Dosis zwischen 1 mg/kg bis 100 mg/kg beim Menschen beträgt.

## Claims

1. Compounds of following general formula (I): 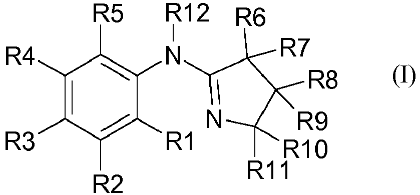 wherein:
a) either R12 represents H, and
- R1 and R2 each independently are:
a halogen, straight-chain or branched C1 to C8 alkyl, C2-C8 alkene, straight-chain or branched C1 to C8 alkoxy, C3-C6 cycloalkyl, C5-C6 bicycloalkyl, a polyether chain, C1-C5 perfluoroalkyl, C1-C8 acyl, OH, SH, a primary, secondary or tertiary amine, CN, CO₂H, CO₂R' where R' is a straight-chain or branched C1-C8 alkyl or C3-C6 cycloalkyl; or
R1 and R2 together form a C5 ring
- R3, R4 and R5 each independently represent:
a hydrogen, a halogen, straight-chain or branched C1 to C8 alkyl, C2-C8 alkene, straight-chain or branched C1 to C8 alkoxy, C3-C6 cycloalkyl, C5-C6 bicycloalkyl, a polyether chain, a C1-C5 perfluoroalkyl, C1-C8 acyl, OH, SH, a primary, secondary or tertiary amine, CN, CO₂H, CO₂R' where R' is a straight-chain or branched C1-C8 alkyl, or a C3-C6 cycloalkyl;
- R6, R7, R9 and R11 represent hydrogens;
- R8 and R10 are chosen from the group consisting of a hydrogen and straight-chain or branched C1 to C5 alkyl, at least one of the two being a straight-chain or branched C1 to C5 alkyl;
b) or R12 represents CH(R13)(CH₂) and forms a C5 ring with R5, R13 representing H or CH₃,
- R1, R2, R3 and R4 are chosen from the group consisting of a hydrogen, a halogen, straight-chain or branched C1 to C8 alkyl, C2-C8 alkene, straight-chain or branched C1 to C8 alkoxy, C3-C6 cycloalkyl, C5-C6 bicycloalkyl, a polyether chain, a C1-C5 perfluoroalkyl, C1-C8 acyl, -OH, -SH, a primary, secondary or tertiary amine, -CN, -CO₂H, -CO₂R' where R' is a straight-chain or branched C1-C8 alkyl, and a C3-C6 cycloalkyl;
- R6, R7, R9 and R11 represent hydrogens;
- R8 and R10 are chosen from the group consisting of a hydrogen and straight-chain or branched C1 to C5 alkyl, at least one of the two being a straight-chain or branched C1 to C5 alkyl,
provided that at least one from among R1 and R13 is not a hydrogen;
and the pharmacologically acceptable salts thereof.

2. The compounds according to claim 1 of following general formula (Ia): 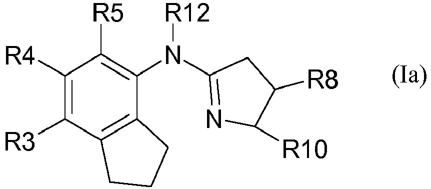 wherein:
R3 and R12 are hydrogens;
R8 and R10 are chosen from the group consisting of a hydrogen and straight-chain or branched C1 to C5 alkyl, at least one of the two being a straight-chain or branched C1 to C5 alkyl; and
R4 and R5 are independently chosen from the group consisting of a hydrogen, halogen, straight-chain or branched C1 to C3 alkyl, straight-chain or branched C1 to C3 alkoxy, C1-C3 perfluoroalkyl, C1-C3 acyl, OH, SH, a primary, secondary or tertiary amine, CN, CO₂H and CO₂R' where R' is a straight-chain or branched C1-C3 alkyl, preferably from the group consisting of a halogen, straight-chain or branched C1 to C3 alkyl, straight-chain or branched C1 to C3 alkoxy, C1-C3 perfluoroalkyl and a C1-C3 acyl.

3. The compounds according to claim 2 wherein R4 and R5 are hydrogens.

4. The compounds according to claim 1 of following general formula (Ib): 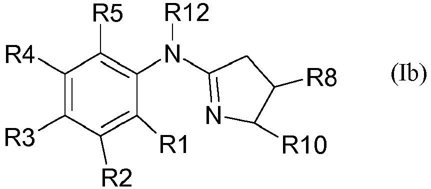 where:
R12 is a hydrogen;
R8 and R10 are chosen from the group consisting of a hydrogen and straight-chain or branched C1 to C5 alkyl, at least one of the two being a straight-chain or branched C1 to C5 alkyl;
R1 and R2 are independently chosen from the group consisting of a halogen, straight-chain or branched C1 to C3 alkyl, straight-chain or branched C1 to C3 alkoxy, C1-C3 perfluoroalkyl and a C1-C3 acyl; and
R3, R4 and R5 are independently chosen from the group consisting of a hydrogen, halogen, straight-chain or branched C1 to C3 alkyl, straight-chain or branched C1 to C3 alcoxy, C1-C3 perfluoroalkyl, C1-C3 acyl, OH, SH, a primary, secondary or tertiary amine, CN, CO₂H and CO₂R' where R' is a straight-chain or branched C1-C3 alkyl, preferably from the group consisting of a halogen, straight-chain or branched C1 to C3 alkyl, straight-chain or branched C1 to C3 alkoxy, C1-C3 perfluoroalkyl and a C1-C3 acyl.

5. The compounds according to claim 4 wherein R3, R4 and R5 are hydrogens.

6. The compounds according to claim 4 or 5 wherein R1 and R2 are independently chosen from the group consisting of a halogen and straight-chain or branched C1 to C3 alkyl.

7. The compound according to one of claims 1 to 6 chosen from among one of the following formulas:

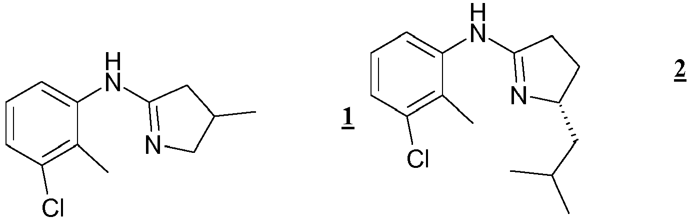

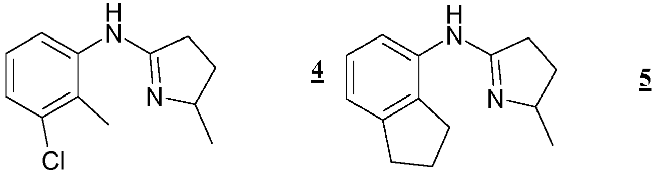

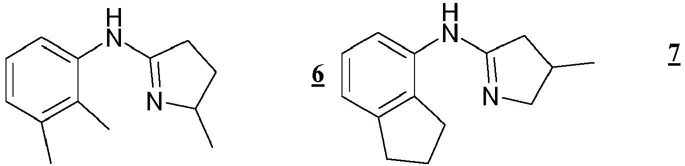

8. The compounds according to claim 1 of following general formula (Ic): 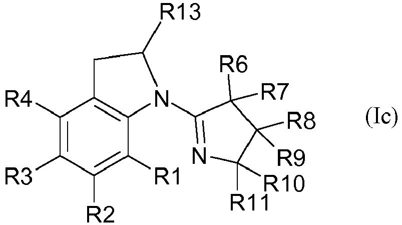 wherein:
- R1, R2, R3 and R4 are chosen from the group consisting of a hydrogen, halogen, straight-chain or branched C1 to C3 alkyl, straight-chain or branched C1 to C3 alkoxy, C1-C3 perfluoroalkyl, C1-C3 acyl, OH, SH, a primary, secondary or tertiary amine, -CN, -CO₂H and -CO₂R' where R' is a straight-chain or branched C1-C3 alkyl;
- R8 and R10 are chosen from the group consisting of a hydrogen and straight-chain or branched C1 to C5 alkyl, at least one of the two being a hydrogen, provided that at least one from among R1 and R13 is not a hydrogen.

9. The compounds according to claim 8 wherein:
- R2, R3, R4 are hydrogens,
- R13 is H and R1 is a halogen and straight-chain or branched C1 to C3 alkyl; or R13 is a methyl and R1 is chosen from the group consisting of a hydrogen, halogen and straight-chain or branched C1 to C3 alkyl; and
- R8 and R10 are chosen from the group consisting of a hydrogen and straight-chain or branched C1 to C5 alkyl, at least one of the two being a hydrogen.

10. The compounds according to claim 8 or 9 chosen from among one of the following formulas:

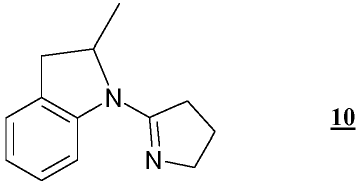

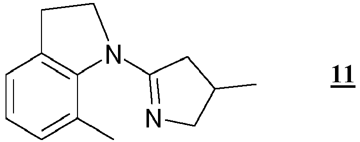

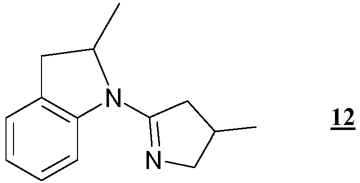

11. The compounds such as defined in one of claims 1 to 10 for use in the prevention and/or treatment of metabolic syndrome.

12. A pharmaceutical composition comprising as active ingredient at least one compound according to one of claims 1 to 10 in association with a pharmaceutically acceptable vehicle.

13. A pharmaceutical composition according to claim 12 which can be administered via oral route.

14. A pharmaceutical composition which can be administered via oral route according to claim 13 wherein the dose of the active ingredient is between 1 mg/kg and 100 mg/kg in man.
